(19)

Europäisches
Patentamt
European
Patent Office
Office européen
des brevets

(11) **EP 3 926 640 A1**

(12) **EUROPEAN PATENT APPLICATION**
published in accordance with Art. 153(4) EPC

(43) Date of publication:
**22.12.2021 Bulletin 2021/51**

(21) Application number: **19914995.6**

(22) Date of filing: **12.02.2019**

(51) Int Cl.:
**G16H 40/20** (2018.01)  **G06Q 10/02** (2012.01)
**G06Q 10/08** (2012.01)

(86) International application number:
**PCT/JP2019/004830**

(87) International publication number:
**WO 2020/165940 (20.08.2020 Gazette 2020/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Medipal Holdings Corporation
Tokyo 104-8461 (JP)**

(72) Inventor: **NAGASAWA Kazunori
Tokyo 104-8461 (JP)**

(74) Representative: **Stöckeler, Ferdinand et al
Schoppe, Zimmermann, Stöckeler
Zinkler, Schenk & Partner mbB
Patentanwälte
Radlkoferstrasse 2
81373 München (DE)**

(54) **SCHEDULE CALCULATING DEVICE, SCHEDULE MANAGEMENT SYSTEM, AND SCHEDULE CALCULATING METHOD**

(57) A transportation schedule is calculated for a regenerative medicine process related to autologous cells, which is related to a first container used for accommodating a collected material collected from a human body and a second container used for accommodating a drug produced using the collected material. A received-order management server 11 (a schedule calculation device) includes a reception unit 30a that receives administration date data related to a drug specified by a user with respect to calendar screen data for each user displayed on a client terminal 5 (a user terminal) provided in a medical facility 31, from the client terminal 5 (the user terminal), a schedule calculation unit 30b that calculates each of execution dates on which a plurality of processes related to transportation from a collection date to an administration date are respectively performed based on the administration date data received by the reception unit 30a, and a calendar-screen generation unit 30d that generates calendar screen data including the execution dates from the collection date to the administration date.

**FIG.1**

EP 3 926 640 A1

**Description**

Field

The present invention relates to a schedule calculation device, a schedule management system, and a schedule calculation method that are suitable for calculating a transportation schedule for a regenerative medicine process related to autologous cells which is for transporting a collected material collected from the body of a patient in a medical facility to a culture facility and for transporting a drug cultured from the collected material in the culture facility to the medical facility.

Background

In recent years, autologous cell transplantation has been performed in which cells collected from the body of a patient are cultured and then transplanted into the body of the patient. In order to perform this autologous cell transplantation, a cell culture center (hereinafter, "culture facility") that expertly cultures the collected cells is required.

However, since the number of culture facilities for culturing cells is relatively small with respect to the number of medical facilities where patients are hospitalized, it is necessary to manage routes and schedules of transportation between those facilities.

Patent Literature 1 discloses a technique that aims to execute a transportation plan or a culture plan in order to efficiently transport radiopharmaceuticals. The technique memorizes location data indicating locations of transportation bases of radioactive materials and locations of a plurality of inspection facilities that perform inspection using radioactive materials, memorizes inspection schedule data including times of inspection performed in the respective inspection facilities, and generates transportation routes for one or more transportation vehicles to transport radioactive materials from the transportation bases to one or more inspection facilities and a transportation schedule that includes the time of delivery to each inspection facility based on the location data and the inspection schedule data.

In Patent Literature 1, transportation of radiopharmaceuticals requires packing in accordance with a prescribed packaging standard for radioactive materials and, in some cases, requires a dedicated transportation vehicle suitable for radioactive materials. Therefore, handling of radiopharmaceuticals is different from that of a general cargo.

Citation List

Patent Literature

Patent Literature 1: Japanese Patent No. 6275388

Summary

Technical Problem

In Patent Literature 1, a product container for transportation is placed in a cardboard box constituting a cushioning material. However, Patent Literature 1 fails to disclose the way of handling the product container for transportation after being transported.

Further, in autologous cell transplantation, different dedicated containers have to be used as a first container used exclusively for transporting cells collected from the body of a patient in a medical facility to a culture facility and a second container used exclusively for transporting a drug cultured in the culture facility to the medical facility where the patient is present, because the first and second containers are different in storage capacity, storage temperature, storage humidity, and the like.

Therefore, there has been a demand for calculating a transportation schedule for a regenerative medicine process related to autologous cells.

An embodiment of the present invention has been made in view of the above problems, and an object of the embodiment is to calculate a transportation schedule related to a first container used for accommodating a collected material collected from a human body and a second container used for accommodating a drug produced using the collected material, for a regenerative medicine process related to autologous cells.

Solution to Problem

In order to solve the above problem, the invention according to claim 1 is a schedule calculation device for calculating a transportation schedule for a regenerative medicine process related to autologous cells, the transportation schedule including a first transportation process that accommodates a collected material collected from a human body in a first container in a medical facility and thereafter transports the first container to a culture facility and a second transportation process that accommodates a drug produced using the collected material in a second container different from the first container in a culture facility and thereafter transports the second container to the medical facility, the device comprising: a reception unit that receives administration date data related to the drug specified by a user with respect to calendar screen data for each user displayed on a user terminal provided in the medical facility, from the user terminal; a schedule calculation unit that calculates each of execution dates on which a plurality of processes related to transportation from a collection date to the administration date are respectively performed based on the administration date data received by the reception unit; and a calendar-screen generation unit that generates calendar screen data including the execution dates from the collection date to the administration date.

Advantageous Effects of Invention

According to the present invention, it is possible to calculate a transportation schedule related to a first container used for accommodating a collected material collected from a human body and a second container used for accommodating a drug produced using the collected material, for a regenerative medicine process related to autologous cells.

Brief Description of Drawings

[FIG. 1] FIG. 1 is a block diagram illustrating a configuration of a schedule management system according to a first embodiment of the present invention.
[FIG. 2] FIG. 2 is a diagram illustrating a vehicle on which a transportation unit according to the first embodiment of the present invention is placed.
[FIG. 3] FIG. 3 is a diagram illustrating a hardware configuration of the transportation unit according to the first embodiment of the present invention.
[FIG. 4] FIG. 4 is a functional block diagram of a received-order management server according to the first embodiment of the present invention.
[FIG. 5] FIG. 5 is a sequence diagram illustrating a flow of a material according to the first embodiment of the present invention.
[FIG. 6] FIG. 6 is a sequence diagram illustrating a procedure from delivery of an empty container A to delivery of cells.
[FIG. 7] FIG. 7 is a sequence diagram illustrating a procedure from collection of a processed item to administration.
[FIGS. 8] FIG. 8(a) is a diagram illustrating a calendar screen generated by the received-order management server according to the first embodiment of the present invention in a case where the number of times of collection is one, and FIG. 8(b) is a table illustrating mark data, signs, and names displayed on the calendar screen.
[FIG. 9] FIG. 9 is a sequence diagram used when a calendar screen in a case where the number of times of collection is one is displayed.
[FIG. 10] FIG. 10 is a comparison table for calculation of calculated dates in a case where the number of times of collection is one.
[FIGS. 11] FIG. 11(a) is a schedule calculation flowchart used by the received-order management server, and FIG. 11(b) is a diagram illustrating details of a calculation file.
[FIG. 12] FIG. 12 is an ER diagram illustrating configurations and association of files and masters to be managed by the received-order management server according to the first embodiment of the present invention.
[FIG. 13] FIG. 13 is a diagram illustrating a calendar screen in a case where the number of times of collection is more than one.
[FIG. 14] FIG. 14 is a sequence diagram used when a calendar screen in a case where the number of times of collection is more than one is displayed.
[FIG. 15] FIG. 15 is a comparison table for calculation of calculated dates in a case where the number of times of collection is more than one.
[FIG. 16] FIG. 16 is a flowchart illustrating a process of generating a calendar screen by a received-order management server according to a third embodiment of the present invention.
[FIG. 17] FIG. 17 is a flowchart illustrating a sub-routine of a mark display process by the received-order management server according to the third embodiment of the present invention.
[FIG. 18] FIG. 18 is a comparison table representing association between event codes and first mark data used by the received-order management server according to the third embodiment of the present invention in a mark display process.
[FIG. 19] FIG. 19 is a comparison table representing association between the number of events and second mark data used by the received-order management server according to the third embodiment of the present invention in the mark display process.

[FIG. 20] FIG. 20 is a comparison table that is an example of the result of the mark display process performed by the received-order management server according to the third embodiment of the present invention, and that represents mark data obtained by combining an event code and the number of events.

[FIG. 21] FIG. 21 is an ER diagram illustrating configurations and association of a management calendar file and a management-calendar event file to be managed by the received-order management server according to the third embodiment of the present invention.

Description of Embodiments

The present invention is explained below in more detail by embodiments illustrated in the drawings.

The present invention has the following configuration in order to calculate a transportation schedule related to a first container used for accommodating a collected material collected from a human body and a second container used for accommodating a drug produced using the collected material, for a regenerative medicine process related to autologous cells.

That is, a schedule calculation device according to the present invention is a schedule calculation device that calculates a transportation schedule for a regenerative medicine process related to autologous cells, the transportation schedule including a first transportation process of accommodating a collected material collected from a human body in a first container in a medical facility and thereafter transporting the first container to a culture facility and a second transportation process of accommodating a drug produced using the collected material in a second container different from the first container in the culture facility and thereafter transporting the second container to the medical facility. The schedule calculation device includes a reception unit that receives administration date data related to a drug specified by a user with respect to calendar screen data for each user displayed on a user terminal provided in the medical facility from the user terminal, a schedule calculation unit that calculates each of execution dates on which a plurality of processes related to transportation from a collection date to an administration date are respectively performed based on the administration date data received by the reception unit, and a calendar-screen generation unit that generates calendar screen data including the execution dates from the collection date to the administration date.

By the configuration described above, it is possible to calculate a transportation schedule related to a first container used for accommodating a collected material collected from a human body and a second container used for accommodating a drug produced using the collected material, for a regenerative medicine process related to autologous cells.

Characteristics of the present invention described above are explained in detail with reference to the drawings mentioned below. Note that, unless otherwise specified, constituent elements, types, combinations, shapes, and relative arrangements thereof described in the following embodiments are not intended to limit the scope of the present invention solely thereto and are only explanatory examples.

Characteristics of the present invention described above are explained below in detail with reference to the drawings.

<First embodiment>

<Schedule management system>

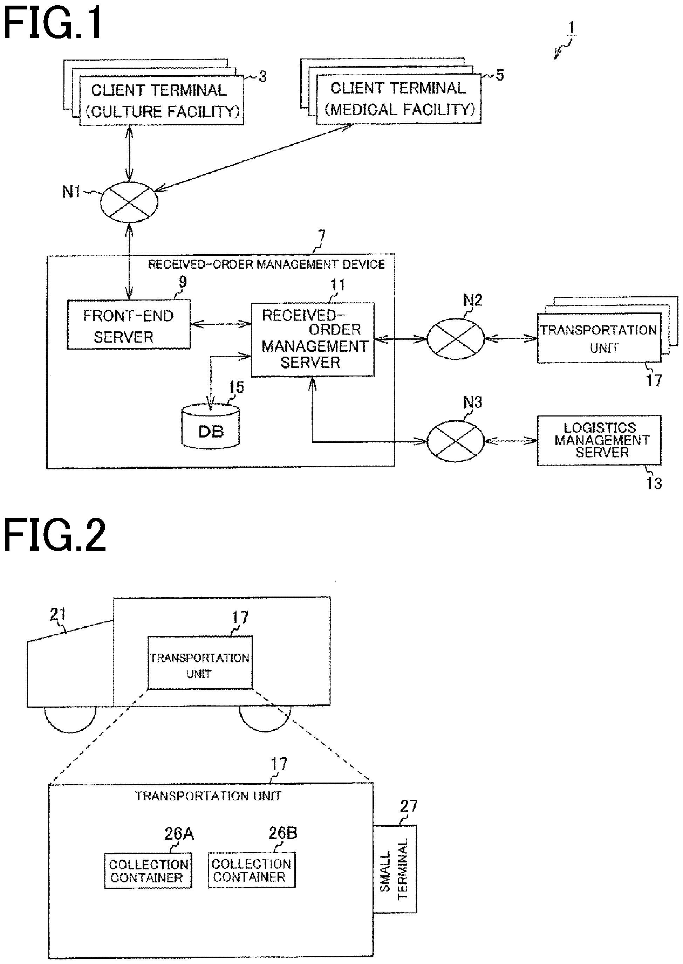

FIG. 1 is a block diagram illustrating a configuration of a schedule management system according to a first embodiment of the present invention.

In the following descriptions, explanations will be made while like constituent elements are denoted by like reference signs.

A schedule management system 1 includes a client terminal (a culture facility) 3, a client terminal (a medical facility) 5, communication networks N1, N2 and N3, a received-order management device 7, a logistics management server (a transporter) 13, a database DB 15, and a transportation unit 17.

The received-order management device 7 includes a front-end server 9 and a received-order management server 11.

In the present embodiment, each of the client terminal 3, the client terminal 5, the transportation unit 17, and the logistics management server 13 is configured in plural. However, each of the elements may be configured in singular. Further, the three separate communication networks N1, N2, and N3 are used. However, these networks may be configured by the same network.

The front-end server 9 has a function of receiving data from the client terminals 3 and 5 via the network N1 to provide a direct access service to the client terminals 3 and 5 and change a display format.

The client terminal 3 is a terminal operable by a staff of a culture facility 35. The client terminal 5 is a terminal operable by a medical staff such as a doctor and a nurse in a medical facility.

The received-order management server 11 receives data of the transportation unit 17 via the communication network N2 to manage the state of each transportation unit 17.

The received-order management server 11 includes a ROM (Read Only Memory), a RAM (Random Access Memory), a CPU (Central Processing Unit), and an HDD (Hard Disk Drive), and reads an operating system OS from the HDD and then loads the OS on the RAM to activate the OS, and reads programs (programs illustrated in various flowcharts described later) from the HDD to perform various types of processing, under control of the OS.

The logistics management server 13 is arranged in each warehouse that stores therein each article or in each warehouse that stores therein a plurality of articles, and executes control for shipping a corresponding article to a client (a medical facility) upon reception of a request from the received-order management server 11.

In the transportation unit 17, a GPS receiver 27e receives each GPS signal from GPS satellites. The transportation unit 17 then calculates location data based on each GPS signal, adds a unique device code to the calculated location data, and transmits the location data to the received-order management server 11 via the communication network N2.

It suffices that the unique device code provided to the GPS receiver 27e is, for example, a MAC address (Media Access Control address) of the GPS receiver 27e or a unique management number set in advance.

<Vehicle with transportation unit placed thereon>

FIG. 2 is a diagram illustrating a vehicle on which the transportation unit according to the first embodiment of the present invention is placed.

A vehicle 21 includes the transportation unit 17 placed on a loading platform thereof. Further, the transportation unit 17 accommodates therein a collection container 26A and a collection container 26B. A small terminal 27 is provided on a side surface of the transportation unit 17 to be in contact therewith.

A part of a tag string fastened to each of the collection containers 26A and 26B accommodated in the transportation unit 17 is pulled outside, and a tag is fastened at the end thereof.

The small terminal 27 is arranged to be in close contact with the side surface of the transportation unit 17 and communicates with the received-order management server 11 via the network N2.

<Transportation unit>

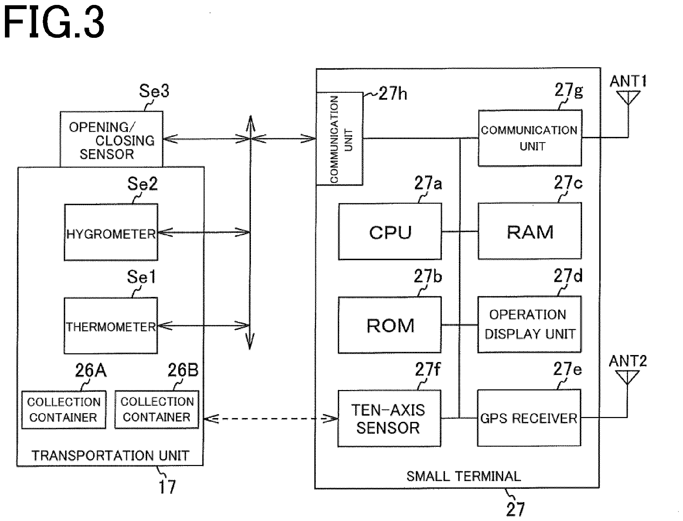

FIG. 3 is a diagram illustrating a hardware configuration of the transportation unit according to the first embodiment of the present invention.

The transportation unit 17 accommodates the collection containers 26A and 26B. The transportation unit 17 includes a plurality of sensors (various sensors Se1 and Se2) described later. A used state/unused state of each sensor can be set from outside.

The transportation unit 17 can accommodate therein a specific type of container from plural types of collection containers prepared in advance according to the type of an article. Also, plural types of the transportation units 17 are prepared which are selectable according to a management condition (temperature, for example).

Here, the type of an article means what is different in accordance with the property, the application, the storage temperature, the storage humidity, or the like of the article. Further, the type of a container means what is different in the storage temperature, the storage humidity, or the like, corresponding to the type of an article.

For example, when a collected material collected from a human body in a medical facility 31 is, for example, peripheral blood, the collection container A (the first container) that accommodates the peripheral blood needs to be maintained at a normal storage temperature.

When the collected material is, for example, bone marrow aspirate, the collection container A (the first container) that accommodates the bone marrow aspirate needs to be maintained at a storage temperature of 15 degrees.

Further, when the collected material is, for example, mucous membranes and/or blood, the collection container A (the first container) that accommodates the mucous membranes and/or blood needs to be maintained at a storage temperature of 4 to 10 degrees.

Meanwhile, the container B (the second container) that accommodates a drug produced using a collected material in the culture facility 35 needs to be maintained at a storage temperature of -170 degrees or lower.

Further, the container B (the second container) that accommodates a cell sheet produced using a collected material in the culture facility 35 needs to be maintained at a storage temperature of 37 degrees.

The transportation unit 17 can accommodate a plurality of articles, and includes the various sensors Se1 and Se2 therein or attached to the outside thereof.

The thermometer Se1 is provided in the transportation unit 17, measures the ambient temperature in the transportation unit 17, and outputs temperature data (T1) to a CPU 27a via a communication unit 27h.

The hygrometer Se2 is provided in the transportation unit 17, measures the ambient humidity in the transportation unit 17, and outputs humidity data (R1) to the CPU 27a via the communication unit 27h.

An opening/closing sensor Se3 is provided on the lid of the transportation unit 17, detects whether the lid of the transportation unit 17 is open or closed, and outputs opening/closing data (C1) to the CPU 27a via the communication unit 27h.

The small terminal 27 includes the CPU 27a, a ROM 27b, a RAM 27c, an operation display unit 27d, a GPS receiver 27e, a ten-axis sensor 27f, a communication unit 27g, and the communication unit 27h.

The CPU 27a controls the entire operation of the transportation unit 17, using the RAM 27c as a work memory, in accordance with a program memorized in advance in the ROM 27b.

The ROM 27b is a read-only non-volatile memory medium and stores therein firmware and various kinds of data.

The RAM 27c is a volatile memory medium capable of high-speed read and write of information and can be used as a work memory.

The operation display unit 27d includes a screen for displaying a menu for various types of setting and mode selection and key buttons, for example, and receives various types of operation requests from a user. For example, a used state/unused state of the plural sensors (the various sensors Se1 to Se3) described later can be set from the operation display unit 27d.

The GPS receiver 27e receives radio signals from a plurality of GPS satellites via an antenna ANT2 to calculate location information of the transportation unit 17, and transmits the calculated location information to the received-order management server 11 via the communication unit 27g.

The ten-axis sensor 27f includes three acceleration sensors for respectively detecting accelerations in the x-, y-, and z- axis directions, three gyro sensors for respectively detecting angular velocities in the x-, y-, and z- axis directions, three electronic compasses for respectively detecting directions in the x-, y-, and z-axis directions with respect to north and south geomagnetism generated by the earth, and one air pressure sensor.

The communication unit 27g has a USB interface, and transmits and receives data to and from the received-order management server 11 via an antenna ANT1 and the communication network N2.

The communication unit 27h receives the temperature data (T1) from the thermometer Se1, the humidity data (R1) from the hygrometer Se2, and the opening/closing data (C1) from the opening/closing sensor Se3, and outputs these pieces of data to the CPU 27a.

<Functional blocks of received-order management server>

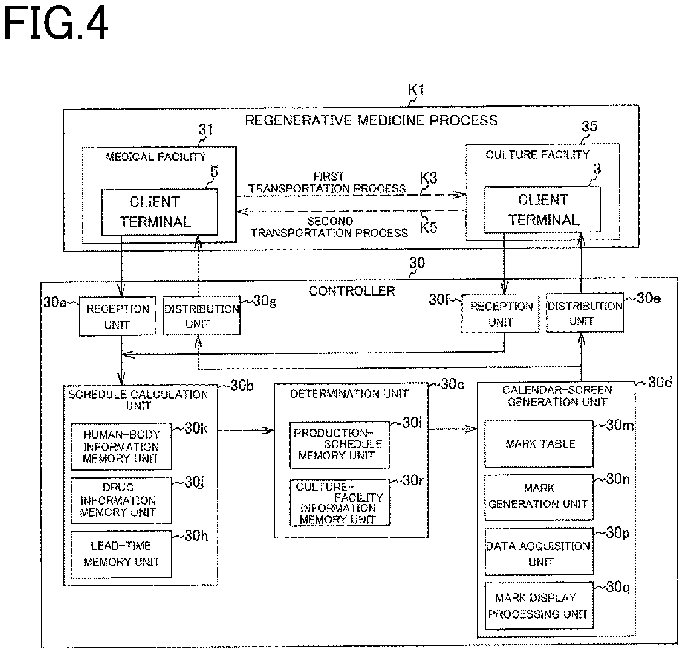

FIG. 4 is a functional block diagram of the received-order management server according to the first embodiment of the present invention.

A regenerative medicine process K1 related to autologous cells includes a first transportation process K3 and a second transportation process K5.

In the first transportation process K3, a collected material collected from a human body in the medical facility 31 is accommodated in the container A (the first container), and thereafter the container A (the first container) is transported to the culture facility 35.

In the second transportation process K5, a drug produced using the collected material in the culture facility 35 is accommodated in the container B (the second container) different from the container A (the first container), and thereafter the container B (the second container) is transported to the medical facility 31.

A controller 30 having a ROM, a RAM, and a CPU is provided in the received-order management server 11 illustrated in FIG. 1. The controller 30 reads out an operating system OS from the ROM and loads it onto the RAM to start up the OS, thereby generating functional modules for reading out programs from the ROM and performing various processes under the control of the OS. Each functional module is generated as a block in the controller 30 illustrated in FIG. 4.

The controller 30 includes a reception unit 30a, a schedule calculation unit 30b, a determination unit 30c, and a calendar-screen generation unit 30d.

The reception unit 30a receives, from the client terminal 5 (a user terminal) provided in the medical facility 31, administration date data related to a drug specified by a user with respect to personal calendar screen data displayed on the client terminal 5 (the user terminal).

The schedule calculation unit 30b calculates each of execution dates on which a plurality of processes related to transportation from a collection date to an administration date are respectively performed based on the administration date data received by the reception unit 30a.

The calendar-screen generation unit 30d generates calendar screen data including the execution dates from the collection date to the administration date.

The schedule calculation unit 30b calculates each of the execution dates from the collection date to the administration date by subtracting lead times each of which indicates a time from a certain execution date to another specific execution date in turn.

The schedule calculation unit 30b includes a lead-time memory unit 30h.

The lead-time memory unit 30h memorizes the lead times respectively indicating times related to a plurality of different processes associated in accordance with a specific combination using a customer code specific to the medical facility 31, a drug ID unique to a drug, and a human-body ID unique to a human body.

The schedule calculation unit 30b calculates each of the execution dates using each of the lead times acquired from the lead-time memory unit 30h by using, as a key, the specific combination using the customer code specific to the medical facility 31, the drug ID unique to the drug, and the human-body ID unique to the human body received by the reception unit 30a.

The determination unit 30c determines whether the culture facility 35 can accept the administration date data related to culturing a drug.

When the determination unit 30c determines that the culture facility 35 can accept the administration date data related to culturing a drug, the calendar-screen generation unit 30d generates calendar screen data.

The determination unit 30c includes a production-schedule memory unit 30i. The production-schedule memory unit 30i memorizes the number of acceptable cultures of drug in the culture facility 35.

The determination unit 30c determines whether there are reservations exceeding the number of acceptable cultures of drug acquired from the production-schedule memory unit 30i. When it is determined that the reservations do not exceed the number of acceptable cultures of drug, the calendar screen generation unit generates calendar screen data.

The schedule calculation unit 30b calculates a processed-product delivery date by subtracting an administration lead time indicating a time from delivery of a drug as a processed product to the medical facility 31 to administration of the drug to a human body from the administration date, calculates a processed-product shipping date by subtracting a culture-facility/customer lead time indicating a time of delivery of a collected material from the medical facility 31 to the culture facility 35 from the processed-product delivery date, and calculates a scheduled process completion date by subtracting a processed-product shipping lead time indicating a time from a process completion date to departure of the processed product from the culture facility 35 from the processed-product shipping date.

The schedule calculation unit 30b includes a drug information memory 30j. The drug information memory 30j memorizes a unit required time required for culturing a drug as a processing lead time.

The schedule calculation unit 30b calculates a collected-material delivery date by subtracting the processing lead time indicating a time from arrival of the collected material at the culture facility 35 to process completion from the scheduled process completion date.

The schedule calculation unit 30b calculates a collected-material shipping date by subtracting the culture-facility/customer lead time from the collected-material delivery date, and calculates the collection date by subtracting a collected-material shipping lead time indicating a time from the collection date of the collected material to departure from the medical facility 31, from the collected-material shipping date.

The schedule calculation unit 30b includes the drug information memory 30j and a human-body information memory unit 30k.

The drug information memory 30j memorizes the number of cells required for culture that are required for culturing a drug.

The human-body information memory unit 30k memorizes the number of collectable cells that can be collected from a human body in one collection, and a collection interval between at least two or more collection dates related to the human body.

The schedule calculation unit 30b sets an integer of a value obtained by dividing the number of cells required for culture acquired from the drug information memory 30j by the number of collectable cells acquired from the human-body information memory unit 30k as the number of times of collection, and calculates the collection dates and the collected-material delivery dates in a case where the number of times of collection is at least two or more based on the number of times of collection and the collection interval acquired from the human-body information memory unit 30k.

The calendar-screen generation unit 30d generates calendar screen data for each user which includes a region for specifying an article, a region for specifying user-identification data, and a region for specifying transportation-destination data.

The calendar-screen generation unit 30d includes a mark table 30m and a mark generation unit 30n.

The mark table 30m memorizes administration-date mark data, processed-product-delivery-date mark data, processed-product-shipping-date mark data, scheduled-process-completion-date mark data, collected-material-delivery-date mark data, collected-material-shipping-date mark data, and collection-date mark data that are image data respectively indicating administration date data, processed-product-delivery date data, processed-product-shipping date data, scheduled-process-completion date data, collected-material-delivery date data, collected-material-shipping date data, and collection date data.

The mark generation unit 30n generates the administration-date mark data, the processed-product-delivery-date mark data, the processed-product-shipping-date mark data, the scheduled-process-completion-date mark data, the collected-material-delivery-date mark data, the collected-material-shipping-date mark data, and the collection-date mark data based on the administration date data, the processed-product-delivery date data, the processed-product-shipping date data, the scheduled-process-completion date data, the collected-material-shipping date data, and the collection date data, referring to the mark table.

The received-order management server 11 (the schedule calculation device) includes a distribution unit 30g.

The distribution unit 30g distributes the administration-date mark data, the processed-product-delivery-date mark data, the processed-product-shipping-date mark data, the scheduled-process-completion-date mark data, the collected-material-delivery-date mark data, the collected-material-shipping-date mark data, and the collection-date mark data that are image data generated by the mark generation unit 30n to a user terminal.

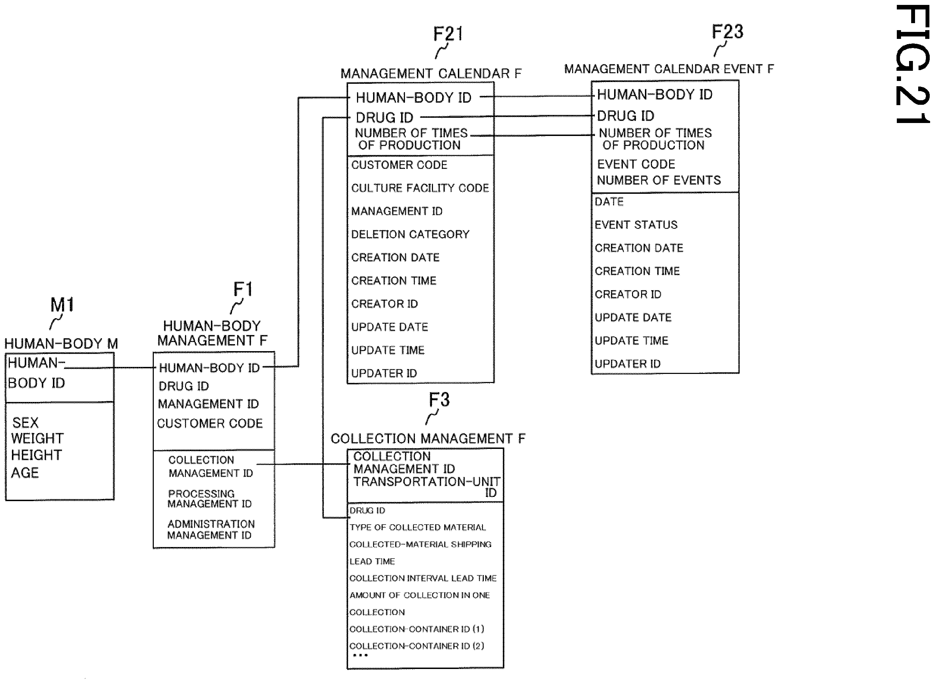

The calendar-screen generation unit 30d includes a management calendar event file F23 (FIG. 21), a data acquisition unit 30p, and a mark display processing unit 30q.

The management calendar event file F23 memorizes a date corresponding to each execution date in association with a human-body ID unique to a human body, a drug ID unique to a drug, an event code, and the number of events.

The data acquisition unit 30p acquires the date corresponding to each execution date, the event code, and the number of events from the management calendar event file, using the human-body ID unique to a human body and the drug ID unique to a drug as a key.

The mark display processing unit 30q superimposes and displays first mark data corresponding to an event code and second mark data corresponding to the number of events in a region corresponding to a date on a calendar screen.

<Sequence diagram representing flow of material>

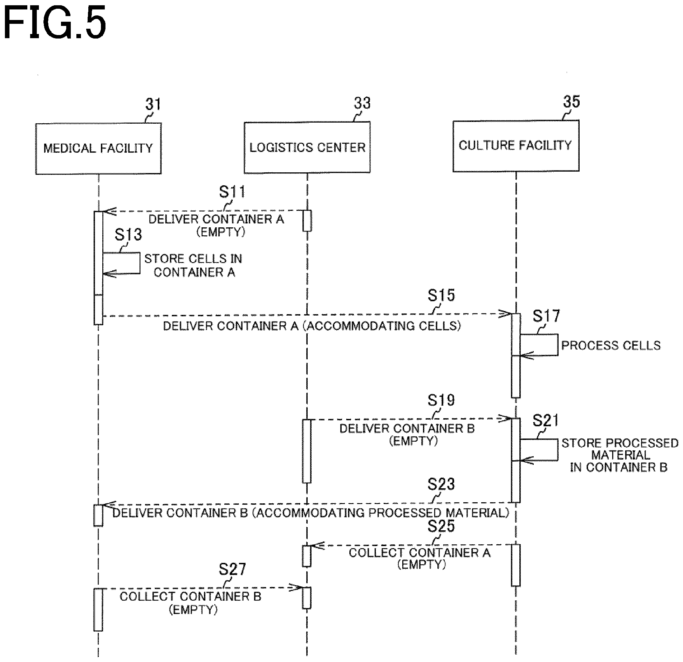

FIG. 5 is a sequence diagram illustrating a flow of a material according to the first embodiment of the present invention.

In the medical facility 31 illustrated in FIG. 5, there is a body of a patient who needs treatment, and a medical staff, for example, collects a specimen such as bone marrow or blood from the body of the patient. A logistics center 33 stocks the transportation unit 17 and the containers (A) and (B). The culture facility 35 processes the specimen into a drug.

At Step S11, a delivery person of the logistics center 33 transports an empty container A and the transportation unit 17 to the medical facility 31 and delivers the empty container A. A medical staff of the medical facility 31 receives the empty container A from a delivery person.

At Step S13, a medical staff of the medical facility 31 stores the specimen collected from the body of the patient in the container A, and further places the container A in the transportation unit 17.

At Step S15, a delivery person transports the transportation unit 17 in which the container A accommodating the specimen is placed in the medical facility 31 to the culture facility 35, and delivers the container A accommodating the specimen.

At Step S17, a staff of the culture facility 35 takes the specimen from the container A and performs culture treatment to process the specimen into a drug.

At Step S19, a delivery person of the logistics center 33 transports the empty container B and the transportation unit 17 to the culture facility 35 and delivers the empty container B.

At Step S21, a staff of the medical facility 35 stores a processed item (the drug) in the container B, and further places the container B in the transportation unit 17.

At Step S23, a delivery person transports the transportation unit 17 in which the container B accommodating the processed item (the drug) is placed to the medical facility 31, and delivers the container B accommodating the processed item.

At Step S25, a delivery person of the logistics center 33 transports the used container A (empty) to the logistics center 33, so that the empty container A is collected.

At Step S27, a delivery person of the logistics center 33 transports the used container B (empty) and the transportation unit 17 to the logistics center 33, so that the empty container B and the transportation unit 17 are collected.

<Sequence diagram from delivery of empty container A to delivery of cells>

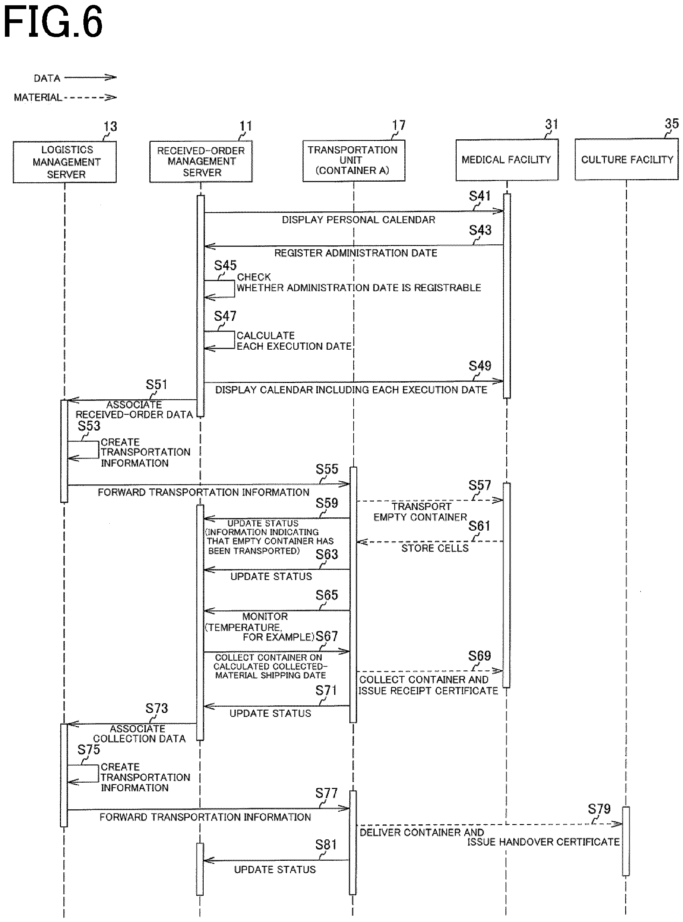

FIG. 6 is a sequence diagram illustrating a procedure from delivery of an empty container A to delivery of cells. Solid arrows illustrated in FIG. 6 indicate flows of data, and broken arrows indicate flows of materials.

At Step S41, the received-order management server 11 transmits screen data of a generated personal calendar to the client terminal 3 in the medical facility 31. Consequently, the client terminal 3 in the medical facility 31 that has received the screen data of the personal calendar from the received-order management server 11 can display an administration-date registration screen of the personal calendar.

At Step S43, the client terminal 3 in the medical facility 31 transmits an administration date specified in accordance with a user's operation with a customer code, a human-body ID, and a drug ID added thereto to the received-order management server 11.

At Step S45, the received-order management server 11 checks (determines) whether the administration date, the customer code, the human-body ID, and the drug ID received from the client terminal 3 are registerable.

That is, the determination unit 30c determines whether the culture facility 35 can accept administration date data related to culturing a drug.

When the determination unit 30c determines that the culture facility 35 can accept the administration date data related to culturing a drug, the calendar-screen generation unit 30d generates calendar screen data.

The determination unit 30c includes a culture-facility information memory unit 30r. The culture-facility information memory unit 30r memorizes the number of acceptable cultures of drug in the culture facility 35.

The determination unit 30c determines whether there are reservations exceeding the number of acceptable cultures of drug acquired from the culture-facility information memory unit 30r. When it is determined that the number of reservations does not exceed the number of acceptable cultures of drug, the process proceeds to Step S47.

At Step S47, the received-order management server 11 calculates each of execution dates on which a plurality of processes related to transportation from the collection date to the administration date are respectively executed based on the administration date.

At Step S49, the received-order management server 11 transmits the personal calendar including the calculated execution dates to the client terminal 3 in the medical facility 31. Consequently, the client terminal 3 in the medical facility 31 that has received screen data of the personal calendar from the received-order management server 11 can display a screen of the personal calendar.

At Step S51, the received-order management server 11 transmits the customer code, the human-body ID, the drug ID, and a calculated collected-material shipping date to the logistics management server 13 in order to perform association of received-order data.

At Step S53, the logistics management server 13 creates transportation information.

At Step S55, the logistics management server 13 forwards the transportation information to the transportation unit 17. The transportation unit 17 memorizes the transportation information received from the logistics management server 13 in a RAM.

At Step S57, a delivery person who has visually confirmed the transportation information places the transportation unit 17 including the empty container A in a vehicle and transports it to the medical facility 31.

At Step S59, the transportation unit 17 transmits, to the received-order management server 11, information indicating that the empty container A has been transported, and updates the status of the transportation unit 17.

At Step S61, human cells are accommodated in the container A placed in the transportation unit 17.

At Step S63, the transportation unit 17 transmits information indicating that the human cells have been accommodated in the container A to the received-order management server 11, and updates the status of the transportation unit 17.

At Step S65, the transportation unit 17 transmits, to the received-order management server 11, monitoring data such as the temperature and the humidity in the container A, the open/closed state of the lid of the container, the vibration transmitted from the environment (road, for example) to the container A, the direction, and the pressure, from a time at which the human cells are accommodated in the container A.

At Step S67, the received-order management server 11 transmits an instruction indicating that the transportation unit 17 including the container A is to be collected from a hospital on the calculated collected-material shipping date, to the transportation unit 17. The transportation unit 17 that has received this collection instruction displays the collection instruction on the operation display unit 27d.

At Step S69, a delivery person who has visually confirmed this collection instruction issues a receipt certificate and passes this certificate to the medical facility 31 when collecting the transportation unit 17 (the container A accommodating the human cells) from the hospital.

At Step S71, the transportation unit 17 transmits information indicating that the container A accommodating the human cells has been collected to the received-order management server 11, and updates the status of the transportation unit 17.

At Step S73, the received-order management server 11 transmits the customer code, the human-body ID, the drug ID, and a calculated collected-material delivery date to the logistics management server 13 in order to perform association of collection data.

At Step S75, the logistics management server 13 creates transportation information.

At Step S77, the logistics management server 13 forwards the transportation information to the transportation unit 17. The transportation unit 17 memorizes the transportation information received from the logistics management server 13 in a RAM.

The transportation unit 17 that has received this transportation instruction displays the transportation instruction on the operation display unit 27d.

At Step S79, a delivery person who has visually confirmed this delivery instruction issues a handover certificate and passes this certificate to the culture facility 35 when delivering the transportation unit 17 (the container A accommodating the human cells).

At Step S81, the transportation unit 17 transmits information indicating that the transportation unit 17 including the container A accommodating the human cells has been delivered to the culture facility 35 to the received-order management server 11, and updates the status of the transportation unit 17.

<Sequence diagram from collection of processed item to administration>

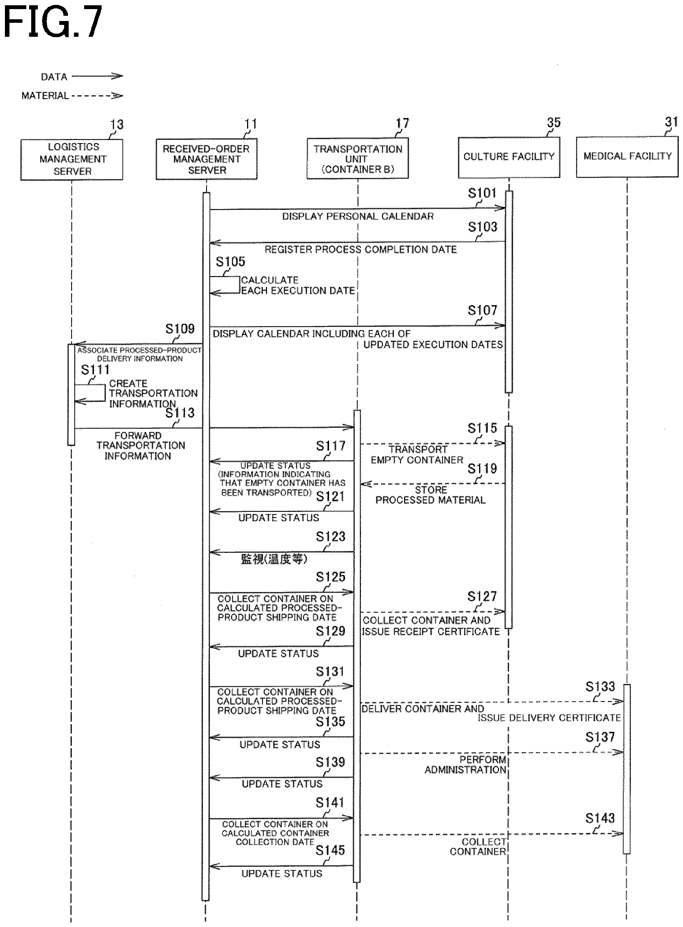

FIG. 7 is a sequence diagram illustrating a procedure from collection of a processed item to administration. Solid arrows illustrated in FIG. 7 indicate flows of data and broken arrows indicate flows of materials.

At Step S101, the received-order management server 11 transmits screen data of a generated personal calendar to the client terminal 3 in the culture facility 35. Consequently, the client terminal 3 in the culture facility 35 that has received the screen data of the personal calendar from the received-order management server 11 can display an administration-date registration screen of the personal calendar.

At Step S103, the client terminal 3 in the culture facility 35 transmits a process completion date specified in accordance with a user's operation with a customer code, a human-body ID, and a drug ID added thereto to the received-order management server 11.

At Step S105, the received-order management server 11 recalculates each of execution dates on which a plurality of processes related to transportation from a collection date to an administration date are performed based on the process completion date.

At Step S107, the received-order management server 11 transmits the personal calendar including the recalculated execution dates to the client terminal 3 in the culture facility 35. Consequently, the client terminal 3 in the culture facility 35 that has received the screen data of the personal calendar from the received-order management server 11 can display a screen of the personal calendar.

At Step S109, the received-order management server 11 transmits the customer code, the human-body ID, the drug ID, and a calculated processed-product shipping date to the logistics management server 13 in order to perform association of processed-product delivery information.

At Step S111, the logistics management server 13 creates transportation information.

At Step S113, the logistics management server 13 forwards the transportation information to the transportation unit 17. The transportation unit 17 memorizes the transportation information received from the logistics management server 13 in a RAM.

At Step S115, a delivery person who has visually confirmed the transportation information places the transportation unit 17 including the empty container B in a vehicle and transports it to the culture facility 35.

At Step S117, the transportation unit 17 transmits, to the received-order management server 11, information indicating that the empty container B has been transported, and updates the status of the transportation unit 17.

At Step S119, a processed item (a drug) is accommodated in the container B placed in the transportation unit 17 in the culture facility 35.

At Step S121, the transportation unit 17 transmits information indicating that the processed item (the drug) has been accommodated in the container B to the received-order management server 11, and updates the status of the transportation unit 17.

At Step S123, the transportation unit 17 transmits, to the received-order management server 11, monitoring data such as the temperature and the humidity in the container B, the open/closed state of the lid of the container, the vibration transmitted from the environment (road, for example) to the container B, the direction, and the pressure from a time at which the processed item (the drug) is accommodated in the container B.

At Step S125, the received-order management server 11 transmits an instruction indicating that the transportation unit 17 including the container B is to be collected from the culture facility 35 on the calculated processed-product shipping date, to the transportation unit 17. The transportation unit 17 that has received this collection instruction displays the collection instruction on the operation display unit 27d.

At Step S127, a delivery person who has visually confirmed this collection instruction issues a receipt certificate and passes this certificate to the culture facility 35 when collecting the transportation unit 17 (the container B accommodating the processed item (the drug)) from the culture facility 35.

At Step S129, the transportation unit 17 transmits information indicating that the container B accommodating the processed item (the drug) has been collected to the received-order management server 11, and updates the status of the transportation unit 17.

At Step S131, the received-order management server 11 transmits an instruction indicating that the transportation unit 17 including the container B is to be delivered on the calculated processed-product shipping date, to the transportation unit 17. The transportation unit 17 that has received this delivery instruction displays the delivery instruction on the operation display unit 27d.

At Step S133, a delivery person who has visually confirmed this delivery instruction issues a delivery certificate and passes this certificate to the medical facility 31 (a hospital), when delivering the transportation unit 17 (the container B accommodating the processed item (the drug)) to the medical facility 31 (the hospital).

At Step S135, the transportation unit 17 transmits information indicating that the container B accommodating the processed item (the drug) has been delivered to the received-order management server 11, and updates the status of the transportation unit 17.

At Step S137, the processed item (the drug) is taken out of the container B placed in the transportation unit 17 and is administered to a human body.

At Step S139, the transportation unit 17 transmits information indicating that the processed item (the drug) has been administered to the body of a patient to the received-order management server 11, and updates the status of the transportation unit 17.

At Step S141, the received-order management server 11 transmits an instruction indicating that the transportation unit 17 including the container B is to be collected from the hospital on a calculated container collection date, to the transportation unit 17. The transportation unit 17 that has received this collection instruction displays the collection instruction on the operation display unit 27d.

At Step S143, a delivery person who has visually confirmed this collection instruction collects the transportation unit 17 (the empty container B) from the medical facility 31.

At Step S145, the transportation unit 17 transmits information indicating that the transportation unit 17 including the empty container B has been collected to the received-order management server 11, and updates the status of the transportation unit 17.

<Calendar screen>

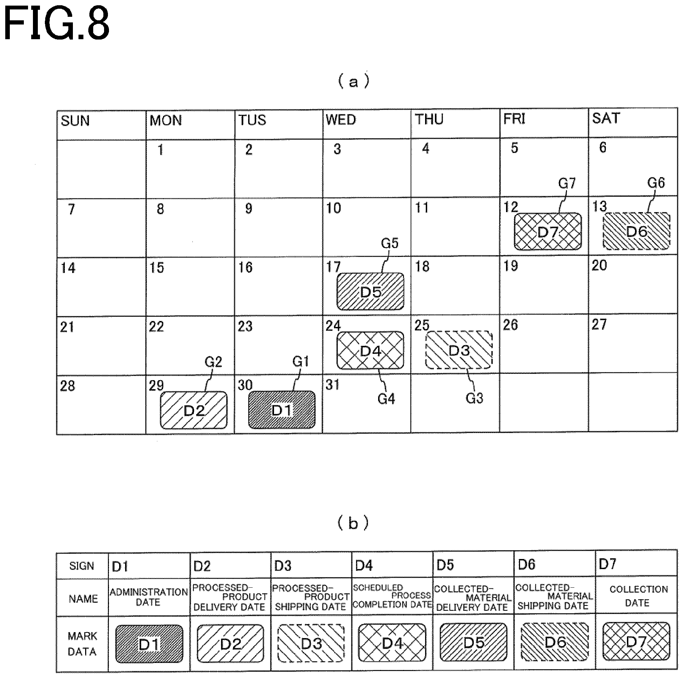

FIG. 8(a) is a diagram illustrating a calendar screen generated by the received-order management server according to the first embodiment of the present invention in a case where the number of times of collection is one.

In a calendar screen generated by the received-order management server 11, seven day-of-the-week regions are arranged in the horizontal direction and a plurality of date regions for each month are arranged in the vertical direction, and the date for each day of the week of a specified month is described in each date region.

In regions of the date regions, which correspond to execution dates, the execution dates denoted by signs D1 to D7 are displayed using mark images G1 to G7, respectively.

In FIG. 8(a), the mark image G1 indicates an administration date D1 by its position on the calendar screen, and the mark image G2 indicates a processed-product delivery date D2 by its position on the calendar screen. Here, an administration lead time T1 (not illustrated) that is a time from the processed-product delivery date D2 to the administration date D1 represents a time from delivery of a processed product to the medical facility 31 to administration to the body of a patient.

The mark image G3 indicates a processed-product shipping date D3 by its position on the calendar screen. Here, a CPC/customer lead time T2 (not illustrated) that is a time from the processed-product shipping date D3 to the processed-product delivery date D2 represents a time taken to deliver a collected material from the medical facility 31 to a CPC (a culture facility) or a time taken to deliver the processed product from the CPC to the medical facility 31.

The mark image G4 indicates a scheduled process completion date D4 by its position on the calendar screen. Here, a processed-product shipping lead time T3 (not illustrated) that is a time from the scheduled process completion date D4 to the processed-product shipping date D3 represents a time from a process completion date to departure of the processed product from the CPC.

The mark image G5 indicates a collected-material delivery date D5 by its position on the calendar screen. Here, a processing lead time T4 (not illustrated) that is a time from the collected-material delivery date D5 to the scheduled process completion date D4 represents a time from arrival of a collected material at the CPC to process completion.

The mark image G6 indicates a collected-material shipping date D6 by its position on the calendar screen. Here, the CPC/customer lead time T2 (not illustrated) that is a time from the collected-material shipping date D6 to the collected-material delivery date D5 represents the time taken to deliver the collected material from the medical facility 31 to the CPC (the culture facility) or the time taken to deliver the processed product from the CPC to the medical facility 31.

The mark image G7 indicates a collection date D7 by its position on the calendar screen. Here, a collected-material shipping lead time T5 (not illustrated) that is a time from the collection date D7 to the collected-material shipping date D6 represents a time from a date of collection of the collected material to departure of the collected material from the medical facility 31.

FIG. 8(b) is a table illustrating mark data, signs, and names displayed on the calendar screen.

As illustrated in FIG. 8(b), image date including the character "D1" represents an administration date and is associated therewith by the sign D1 on a one-to-one basis.

Mark data that is image date including the character "D2" represents a processed-product delivery date that is one of the names, sand is associated therewith by the sign D2 on a one-to-one basis.

Mark data that is image date including the character "D3" represents a processed-product shipping date that is one of the names, and is associated therewith by the sign D3 on a one-to-one basis.

Mark data that is image date including the character "D4" represents a scheduled process completion date that is one of the names, and is associated therewith by the sign D4 on a one-to-one basis.

Mark data that is image date including the character "D5" represents a collected-material delivery date that is one of the names, and is associated therewith by the sign D5 on a one-to-one basis.

Mark data that is image date including the character "D6" represents a collected-material shipping date that is one of the names, and is associated therewith by the sign D6 on a one-to-one basis.

Mark data that is image date including the character "D7" represents a collection date that is one of the names, and is associated therewith by the sign D7 on a one-to-one basis.

The mark data, the signs, and the names are associated with each other and are stored in the database DB.

<Sequence diagram used when calendar screen is displayed>

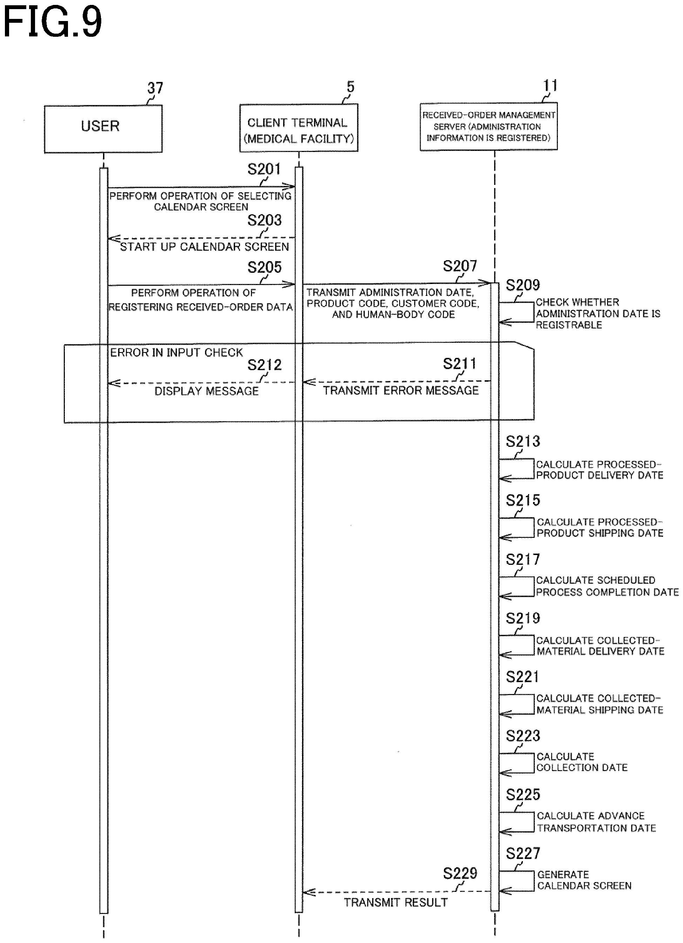

FIG. 9 is a sequence diagram used when a calendar screen in a case where the number of times of collection is one is displayed.

At Step S201, a user (a medical staff) 37 selects an icon of a program for generating a calendar screen which is displayed on the client terminal (the medical facility) 5, in response to a mouse operation.

At Step S203, the client terminal (the medical facility) 5 starts up the program for generating a calendar screen. Consequently, a calendar screen in an initial state is displayed on the client terminal (the medical facility) 5.

At Step S205, the user 37 registers received-order data in the client terminal (the medical facility) 5. At this time, administration date data, a product code, a customer code, a human-body code, and the like are generated by selection in accordance with a mouse operation or input from a keyboard, and are memorized in an internal memory.

At Step S207, the client terminal (the medical facility) 5 transmits the administration date data, the product code, the customer code, the human-body code, and the like to the received-order management server 11.

At Step S209, the received-order management server 11 checks a value of availability of registration of an administration date with regard to each of the administration date data, the product code, the customer code, the human-body code, and the like received from the client terminal (the medical facility) 5.

When the administration date data is abnormal as a result of reception of the administration date data, the product code, the customer code, the human-body code, and the like from the client terminal (the medical facility) 5 and check whether the administration date is registerable with regard to the administration date data at Step S209, the received-order management server 11 transmits an error message to the client terminal (the medical facility) 5 at Step S211.

Consequently, the error message is displayed on the client terminal (the medical facility) 5 at Step S212.

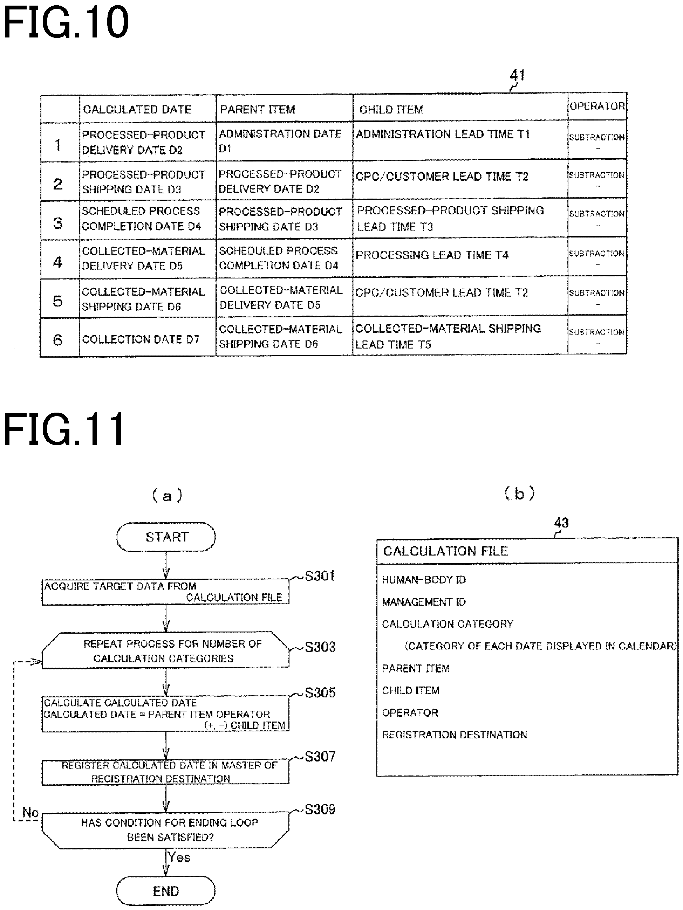

At Step S213, the received-order management server 11 calculates a processed-product delivery date using a schedule calculation flowchart (FIGS. 11).

At Steps S215 to S225, the received-order management server 11 calculates a processed-product shipping date, a scheduled process completion date, a collected-material delivery date, a collected-material shipping date, a collection date, and an advance shipping date in turn. At Step S227, the received-order management server 11 generates screen data of the calendar screen (FIGS. 8) based on each of the results of calculations at Steps S213 to S225, using the schedule calculation flowchart (FIGS. 11) .

At Step S229, the received-order management server 11 transmits the screen data of the calendar screen (FIGS. 8) calculated at Step S227 to the client terminal (the medical facility) 5.

Consequently, the screen data of the calendar screen (FIGS. 8) is displayed on the client terminal (the medical facility) 5.

<Comparison table for calculation of calculated dates>

FIG. 10 is a comparison table for calculation of calculated dates in a case where the number of times of collection is one, which is referred to at Step S305 described later.

In a comparison table 41 illustrated in FIG. 10, a record number, a calculated date, a parent item, a child item, and an operator are described in this order as items arranged in the horizontal direction.

More specifically, when the record number is 1, the processed-product delivery date D2, the administration date D1, the administration lead time T1, and subtraction "-" are described in the order of the calculated date, the parent item, the child item, and the operator.

When the record number is 2, the processed-product shipping date D3, the processed-product delivery date D2, the CPC/customer lead time T2, and subtraction "-" are described.

When the record number is 3, the scheduled process completion date D4, the processed-product shipping date D3, the processed-product shipping lead time T3, and subtraction "-" are described.

When the record number is 4, the collected-material delivery date D5, the scheduled process completion date D4, the processing lead time T4, and subtraction "-" are described.

When the record number is 5, the collected-material shipping date D6, the collected-material delivery date D5, the CPC/customer lead time T2, and subtraction "-" are described.

When the record number is 6, the collection date D7, the collected-material shipping date D6, the collected-material shipping lead time T5, and subtraction "-" are described.

<Schedule calculation flowchart>

FIG. 11(a) is a schedule calculation flowchart used by the received-order management server, and FIG. 11(b) is a calculation file referred to by the schedule calculation flowchart.

In a calculation file 43 illustrated in FIG. 11(b), indices (headings) are memorized which respectively represent a human-body ID, a management ID, a calculation category indicating a category of each date displayed on a calendar screen, a parent item, a child item, an operator, and a registration destination, for example. As the human-body ID, a human-body ID unique to a patient may be used.

At Step S301, the received-order management server 11 acquires target data from the calculation file 43.

At step S303, the received-order management server 11 repeats the process illustrated at the following Step S305 for the number of calculation categories.

At step S305, the received-order management server 11 refers to the comparison table illustrated in FIG. 10 in the order of records, and constructs a calculation formula of a calculated date using a basic arithmetic expression (1) used to construct the calculation formula of the calculated date.

$$\text{Calculated date} = \text{parent item operator} \ (+, \ -) \ \text{child item} \quad (1)$$

At Step S307, the received-order management server 11 registers the calculated date to a master of the registration destination.

At step S309, the received-order management server 11 returns to Step S303 when it has not repeated the process illustrated at the above Step S305 for the number of calculation categories, and ends processing when it has repeated the process illustrated at the above Step S305 for the number of calculation categories.

Here, the arithmetic process at Step S305 is described.

More specifically, in a case where the record number is 1 in the comparison table illustrated in FIG. 10, when the calculation formula is constructed in accordance with the above expression (1) in the order of the calculated date, the parent item, the child item, and the operator, the following expression (2) is obtained.

$$D2 = D1 - T1 \quad (2)$$

Therefore, the administration lead time T1 is subtracted from the administration date D1 to obtain the processed-product delivery date D2 in accordance with the expression (2).

Similarly, in a case where the record number is 2, when the calculation formula is constructed in accordance with the above expression (1), the following expression (3) is obtained.

$$D3 = D2 - T2 \quad (3)$$

Therefore, the CPC/customer lead time T2 is subtracted from the processed-product delivery date D2 to obtain the processed-product shipping date D3 in accordance with the expression (3).

Similarly, in a case where the record number is 3, when the calculation formula is constructed in accordance with the above expression (1), the following expression (4) is obtained.

$$D4 = D3 - T3 \quad (4)$$

Therefore, the processed-product shipping lead time T3 is subtracted from the processed-product shipping date D3 to obtain the scheduled process completion date D4 in accordance with the expression (4).

Similarly, in a case where the record number is 4, when the calculation formula is constructed in accordance with the above expression (1), the following expression (5) is obtained.

$$D5=D4-T4 \quad (5)$$

Therefore, the processing lead time T4 is subtracted from the scheduled process completion date D4 to obtain the collected-material delivery date D5 in accordance with the expression (5).

Similarly, in a case where the record number is 5, when the calculation formula is constructed in accordance with the above expression (1), the following expression (6) is obtained.

$$D6=D5-T2 \quad (6)$$

Therefore, the CPC/customer lead time T2 is subtracted from the collected-material delivery date D5 to obtain the collected-material shipping date D6 in accordance with the expression (6).

Similarly, in a case where the record number is 6, when the calculation formula is constructed in accordance with the above expression (1), the following expression (7) is obtained.

$$D7=D6-T5 \quad (7)$$

Therefore, the collected-material shipping lead time T5 is subtracted from the collected-material shipping date D6 to obtain the collection date D7 in accordance with the expression (7).

<ER diagram>

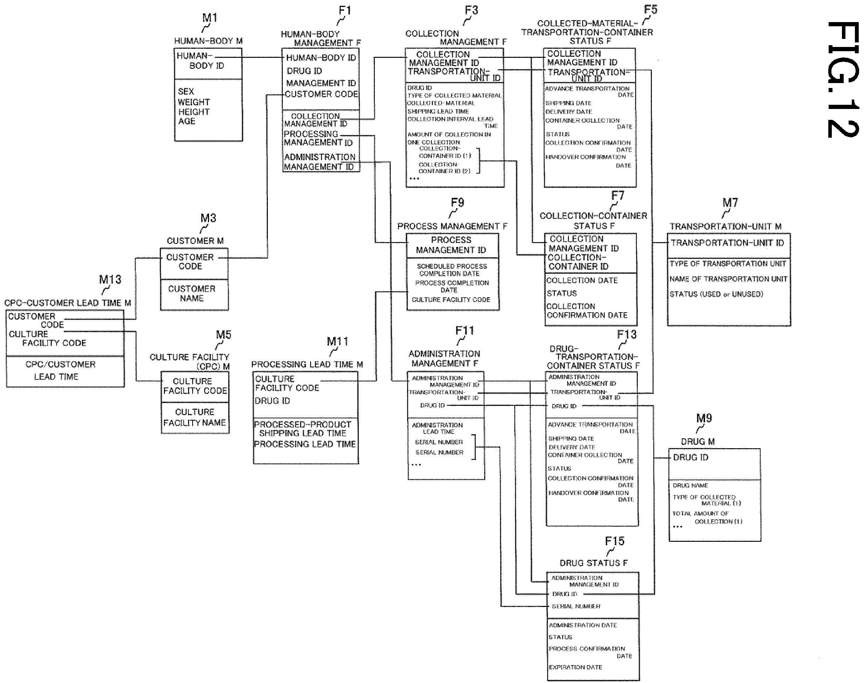

FIG. 12 is an ER diagram illustrating configurations and association of files and masters to be managed by the received-order management server according to the first embodiment of the present invention. In FIG. 12, the sign F denotes a file, and the sign M denotes a master.

<Human-body master>

The received-order management server 11 memorizes the sex, the weight, the height, and the age of a human body in a human-body master M1 in association with a human-body ID.

The received-order management server 11 extracts the sex, the weight, the height, and the age of the human body from the human-body master M1 by using the human-body ID as a key.

<Customer master>

The received-order management server 11 memorizes the name of a customer in a customer master M3 in association with a customer code.

The received-order management server 11 extracts the name of the customer from the customer master M3 by using the customer code as a key.

<Culture-facility master>

The received-order management server 11 memorizes the name of a culture facility (CPC: cell culture center) in a culture-facility master M5 in association with a culture facility code.

The received-order management server 11 extracts the name of the culture facility from the culture-facility (CPC) master M5 by using the culture facility code as a key.

<Human-body management file>

The received-order management server 11 memorizes a collection management ID, a process management ID, and an administration management ID in a human-body management file F1 in association with a human-body ID, a drug ID, a management ID, and a customer code.

The received-order management server 11 extracts the collection management ID, the process management ID, and the administration management ID from the human-body management file F1 by using the human-body ID, the drug ID, the management ID, and the customer code as a key.

<Collection management file>

The received-order management server 11 memorizes a drug ID, the type of a collected material, a collected-material shipping lead time, a collection interval lead time, the amount in one collection, and a collection-container ID in a collection management file F3 in association with a collection management ID and a transportation-unit ID.

The received-order management server 11 extracts the drug ID, the type of a collected material, the collected-material shipping lead time, the collection interval lead time, the amount in one collection, and the collection-container ID from the collection management file F3 by using the collection management ID and the transportation-unit ID as a key.

<Collected-material-transportation-container status file>

In a collected-material-transportation-container status file F5, an advance transportation date, a shipping date, a delivery date, a container collection date, the status, a collection confirmation date, and a handover confirmation date of a transportation unit are memorized in association with a collection management ID and a transportation-unit ID.

The received-order management server 11 extracts the advance transportation date, the shipping date, the delivery date, the container collection date, the status, the collection confirmation date, and the handover confirmation date of the transportation unit from the collected-material-transportation-container status F5 by using the collection management ID and the transportation-unit ID as a key.

<Collection-container status file>

A collection date, the status, and a collection confirmation date of a collected material are extracted in a collection-container status file F7 in association with a collection management ID and a collection-container ID.

The received-order management server 11 extracts the collection date, the status, and the collection confirmation date of the collected material from the collection-container status file F7 by using the collection management ID and the collection-container ID as a key.

<Process management file>

In a process management file F9, a scheduled process completion date, a process completion date, and a culture facility code are memorized in association with a process management ID.

The received-order management server 11 extracts the scheduled process completion date, the process completion date, and the culture facility code from the process management file F9 by using the process management ID as a key.

<Administration management file>

In an administration management file F11, an administration lead time and a serial number are memorized in association with an administration management ID, a transportation-unit ID, and a drug ID.

The received-order management serve 11 extracts the administration lead time and the serial number from the administration management file F11 by using the administration management ID, the transportation-unit ID, and the drug ID as a key.

<Drug-transportation-container status file>

In a drug-transportation-container status file F13, an advance transportation date, a shipping date, a delivery date, a container collection date, the status, a collection confirmation date, and a handover confirmation date of a transportation unit for transporting a drug are memorized in association with an administration management ID, a transportation-unit ID, and a drug ID.

The received-order management server 11 extracts the advance transportation date, the shipping date, the delivery date, the container collection date, the status, the collection confirmation date, and the handover confirmation date of the transportation unit for transporting the drug from the drug-transportation-container status file F13 by using the administration management ID, the transportation-unit ID, and the drug ID as a key.

<Drug status file>

In a drug status file F15, an administration date, the status, a process confirmation date, and an expiration date of a drug are memorized in association with an administration management ID, a drug ID, and a serial number.

The received-order management server 11 extracts the administration date, the status, the process confirmation date, and the expiration date of the drug from the drug status file F15 by using the administration management ID, the drug ID, and the serial number as a key.

<Transportation-unit master>

In a transportation-unit master M7, the type of a transportation unit, the name of the transportation unit, and the status are memorized in association with a transportation-unit ID.

The received-order management server 11 extracts the type of the transportation unit, the name of the transportation unit, and the status from the transportation-unit master M7 by using the transportation-unit ID as a key.

<Drug master>

In a drug master M9, the name of a drug, the type of a collected material, and the total collected amount are memorized in association with a drug ID.

The received-order management server 11 extracts the name of the drug, the type of the collected material, and the total collected amount from the drug master M9 by using the drug ID as a key.

<Processing lead time master>

In a processing lead time master M11, a processed-product shipping lead time and a processing lead time are memorized in association with a culture facility code and a drug ID.

The received-order management server 11 extracts the processed-product shipping lead time and the processing lead time from the processing lead time master M11 by using the culture facility code and the drug ID as a key.

<CPC-customer lead time master>

In a CPC-customer lead time master M13, a lead time between the medical facility 31 and a culture facility is memorized in association with a customer code and a culture facility code.

The received-order management server 11 extracts the lead time between the medical facility 31 and the culture facility from the CPC-customer lead time master M13 by using the customer code and the culture facility code as a key.

Next, management between files or between masters is described referring to the ER diagram illustrated in FIG. 12.

<Association between human-body management file and each management file>

In the human-body management file F1, a customer code, a human-body ID, a drug ID of a drug to be administered are associated with each other and are memorized with a management ID assigned thereto.

In order to administer a target drug to the body of, for example, a patient or a subject, the received-order management server 11 divides the entire process into three processes including a collection process of collecting cells, a processing process of processing the collected cells, and an administration process of administering a processed drug, assigns a collection management ID, a process management ID, and an administration management ID to the three processes, respectively, and manages those IDs on the human-body management file F1.

More specifically, in association of the files, information related to collection is managed using the collection management ID, information related to processing is managed using the process management ID, and information related to administration is managed using the administration management ID, as in the ER diagram illustrated in FIG. 12.

<Association between collection management file, collected-material-transportation-container status file, and collection-container status file>

In the collection management file F3, a collection management ID is associated with a transportation-unit ID used when a collected material is transported, and those IDs are memorized.

The received-order management server 11 associates a collection-container ID of a collection container for accommodating a collected material with the collection management ID and the transportation-unit ID and memorizes them in the collection management file F3 and the collected-material-transportation-container status file F5.

In the collected-material-transportation-container status file F5, an advance transportation date, a shipping date, a delivery date, a container collection date, the status, a collection confirmation date, and a handover confirmation date of the transportation unit are memorized to be associated with the collection management file F3 by using the collection management ID and the transportation-unit ID.

In the collection-container status file F7, a collection date, the status, and a collection confirmation date of a collected material are memorized to be associated with the collection management file F3 by using the collection management ID and the collection-container ID.

<Administration management file, drug-transportation-container status file, and drug status file>

In the administration management file F11, a transportation-unit ID for transporting a drug is associated with an administration management ID, and both the IDs are memorized.

In the administration management file F11, the administration management ID is associated with a drug ID associated with the corresponding administration and the transportation-unit ID for carrying that drug, and a serial number for identifying the drug stored in the corresponding transportation unit and an administration lead time are memorized.

In the drug-transportation-container status file F13, an advance transportation date, a shipping date, a delivery date, a container collection date, the status, a collection confirmation date, and a handover confirmation date of the transportation unit are memorized to be associated with the administration management F by using the administration management ID, the transportation-unit ID, and the drug ID.

In the drug status file F15, an administration date, the status, a process confirmation date, and an expiration date of the drug are memorized by using the administration management ID, the drug ID, and the serial number.

<Second embodiment>

<Calendar screen>

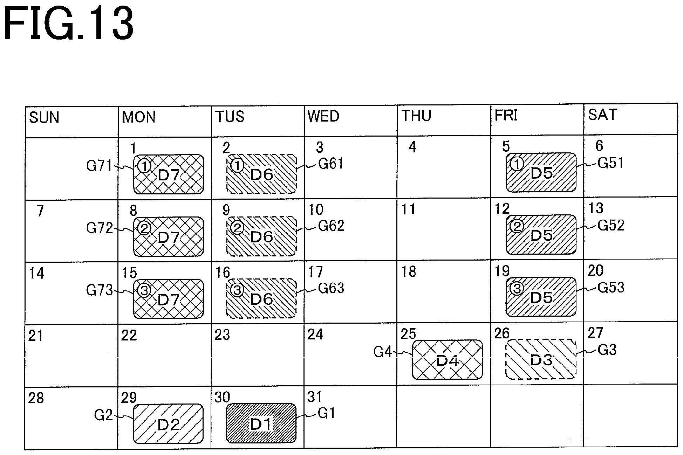

FIG. 13 is a diagram illustrating a calendar screen in a case where the number of times of collection is more than one.

While the calendar screen in a case where the number of times of collection is one has been described referring to FIG. 8(a) in the first embodiment, a calendar screen in a case where the number of times of collection is more than one is described referring to FIG. 13 in a second embodiment. Among the reference signs illustrated in FIG. 13, reference signs same as those illustrated in FIG. 8(a) have the same configuration, and thus descriptions thereof are omitted.

A mark image G53 indicates a collected-material delivery date D5[3] by its position on the calendar screen. Here, the processing lead time T4 (not illustrated) that is a time from the collected-material delivery date D5[3] to the scheduled process completion date D4 represents a time from arrival of a collected material at the culture facility 35 (CPC) to process completion.

A mark image G63 indicates a collected-material shipping date D6[3] by its position on the calendar screen. Here, the CPC/customer lead time T2 (not illustrated) that is a time from the collected-material shipping date D6[3] to the collected-material delivery date D5[3] represents a time taken to deliver the collected material from the medical facility 31 to a CPC (a culture facility). Further, the CPC/customer lead time T2 (not illustrated) represents a time taken to deliver a processed product from the culture facility 35 (the CPC) to the medical facility 31.

A mark image G73 indicates a collection date D73 by its position on the calendar screen. Here, the collected-material shipping lead time T5 (not illustrated) that is a time from the collection date D73 to the collected-material shipping date D63 represents a time from a date of collection of the collected material to departure of the collected material from the medical facility 31.

The collection interval lead time T6 represents a time from the date of collection of the collected material from the body of a patient until a date on which next collection becomes possible.

In the second embodiment, it is necessary to perform collection, for example, three separate times in order to collect a necessary amount of collected material from the body of a patient.

In the calendar screen illustrated in FIG. 13, it is possible to collect a necessary amount of collected material from the body of a patient by securing three collection dates of D71, D72, and D73.

<Sequence diagram used when calendar screen is displayed>

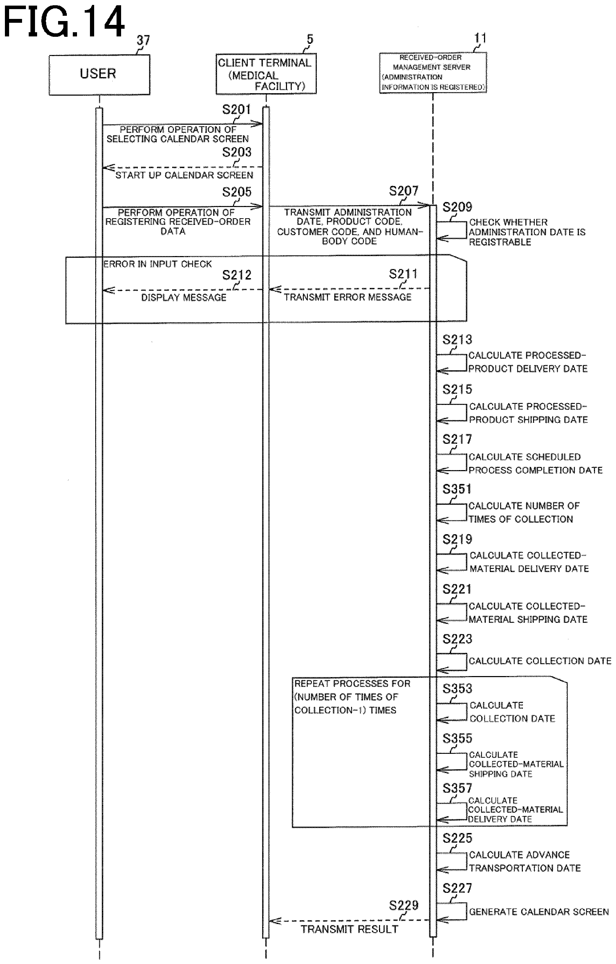

FIG. 14 is a sequence diagram used when a calendar screen in a case where the number of times of collection is more than one is displayed.

In the first embodiment, the sequence diagram in a case where the number of times of collection is one has been described referring to FIG. 9. In the second embodiment, the sequence diagram in a case where the number of times of collection is more than one is described referring to FIG. 14. Among the reference signs illustrated in FIG. 14, reference signs same as those illustrated in FIG. 9 have the same configuration, and thus descriptions thereof are omitted.

At Step S351, the received-order management server 11 calculates the number of times of collection.

At Steps S353 to S357, the received-order management server 11 calculates a collection date, a collected-material shipping date, and a collected-material delivery date in turn.

The received-order management server 11 repeats the processes illustrated at Steps S353 to S357 for (the number of times of collection-1) times.

<Comparison table for calculation of calculated dates>

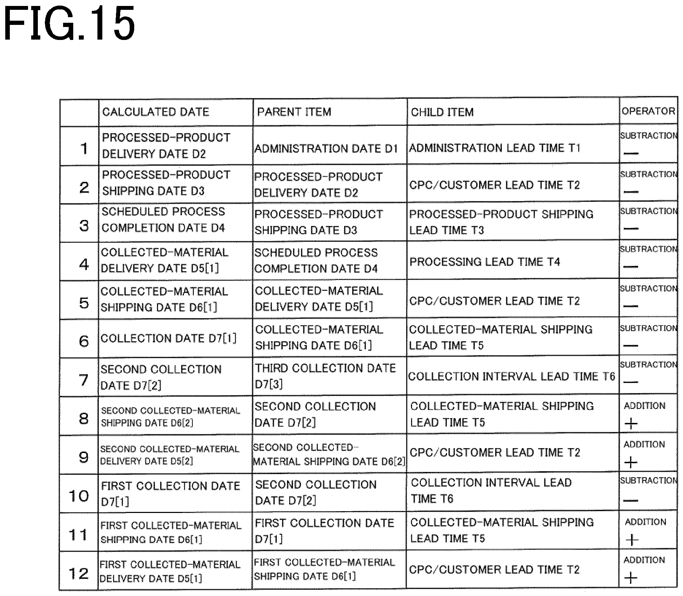

FIG. 15 is a comparison table for calculation of calculated dates in a case where the number of times of collection is more than one, which is referred to at Step S305 illustrated in FIGS. 11.

In the first embodiment, the comparison table for calculation of calculated dates in a case where the number of times of collection is one has been described referring to FIG. 10. In the second embodiment, the comparison table for calculation of calculated tables in a case where the number of times of collection is more than one is described referring to FIG. 15. Among the reference signs illustrated in FIG. 15, reference signs same as those illustrated in FIG. 10 have the same configuration, and thus descriptions thereof are omitted.

When the record number is 4, a collected-material delivery date D5[1], the scheduled process completion date D4, the processing lead time T4, and subtraction "-" are described.

When the record number is 5, a collected-material shipping date D6[1], the collected-material delivery date D5[1], the CPC/customer lead time T2, and subtraction "-" are described.

When the record number is 6, a collection date D7[1], the collected-material shipping date D6[1], the collected-material shipping lead time T5, and subtraction "-" are described.

When the record number is 7, a second collection date D7[2], a third collection date D6[3], the collection interval lead time T6, and subtraction "-" are described.

When the record number is 8, a second collected-material shipping date D6[2], the second collection date D7[2], the collected-material shipping lead time T5, and subtraction "+" are described.

When the record number is 9, a collected-material delivery date D5[2], the second collected-material shipping date D6[2], the CPC/customer lead time T2, and subtraction "+" are described.

When the record number is 10, the first collection date D7[1], the second collection date D6[2], the collection interval lead time T6, and subtraction "-" are described.

When the record number is 11, the first collected-material shipping date D6[1], the first collection date D7[1], the collected-material shipping lead time T5, and subtraction "+" are described.

When the record number is 12, the collected-material delivery date D5[1], the first collected-material shipping date D6[1], the CPC/customer lead time T2, and subtraction "+" are described.

<Third embodiment>

<Process of generating calendar screen>

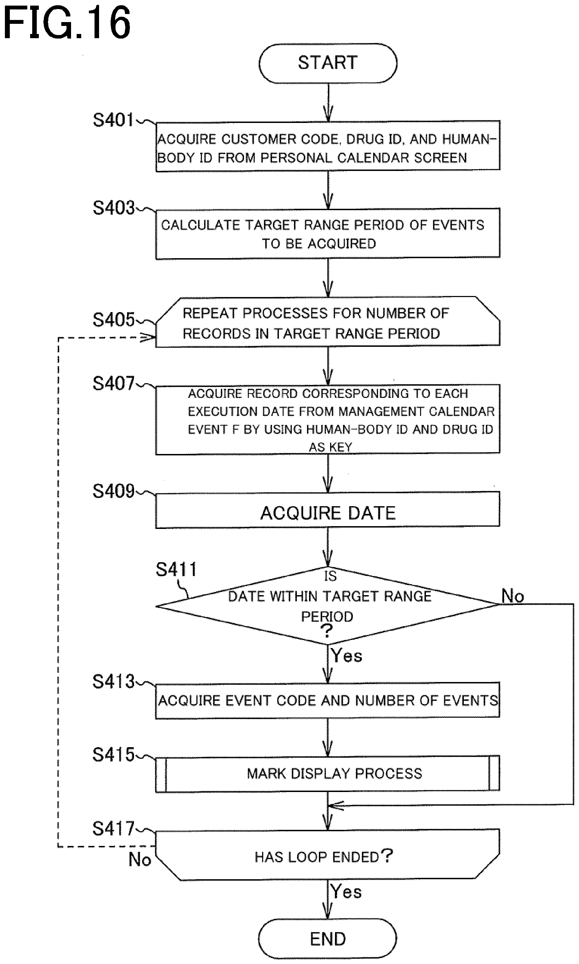

FIG. 16 is a flowchart illustrating a process of generating a calendar screen by the received-order management server according to a third embodiment of the present invention.

At Step S401, the received-order management server 11 acquires a customer code, a drug ID, and a human-body ID from a personal calendar screen displayed on a client terminal.

At Step S403, the received-order management server 11 calculates a target range period of acquired events.

At Step S405, the received-order management server 11 repeats the process for the number of records within the target range period.

At Step S407, the received-order management server 11 acquires a record corresponding to each execution date from the management calendar event F (FIG. 21) by using the human-body ID and the drug ID as a key.

At Step S409, the received-order management server 11 acquires a date from the acquired record.

At step S411, the received-order management server 11 determines whether the date is within the target range period. When the date is within the target range period, the process proceeds to step S413. When the date is not within the target range period, the process proceeds to step S417.

At Step S413, the received-order management server 11 acquires an event code and the number of events.

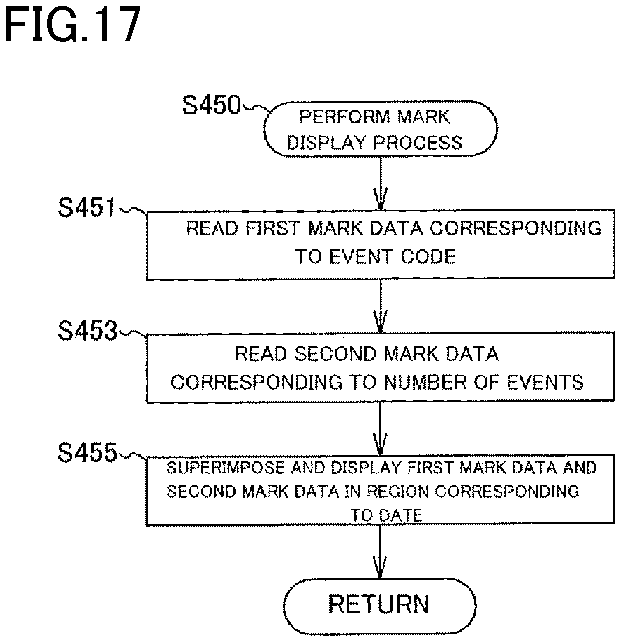

At Step S415, the received-order management server 11 executes a sub-routine (FIG. 17) of a mark display process.

At Step S417, the received-order management server 11 determines whether a loop has ended. When the loop has not ended, the process returns to Step S405. Meanwhile, when the loop has ended, the process is ended.

<Mark display process>

FIG. 17 is a flowchart illustrating a sub-routine of a mark display process by the received-order management server according to the third embodiment of the present invention.
At Step S451, the received-order management server 11 reads first mark data corresponding to an event code.
At Step S453, the received-order management server 11 reads second mark data corresponding to the number of events.
At Step S455, the received-order management server 11 displays the first mark data and the second mark data in a region corresponding to a date on the calendar screen, while the first mark data and the second mark data are superimposed.

<Event code and first mark data>

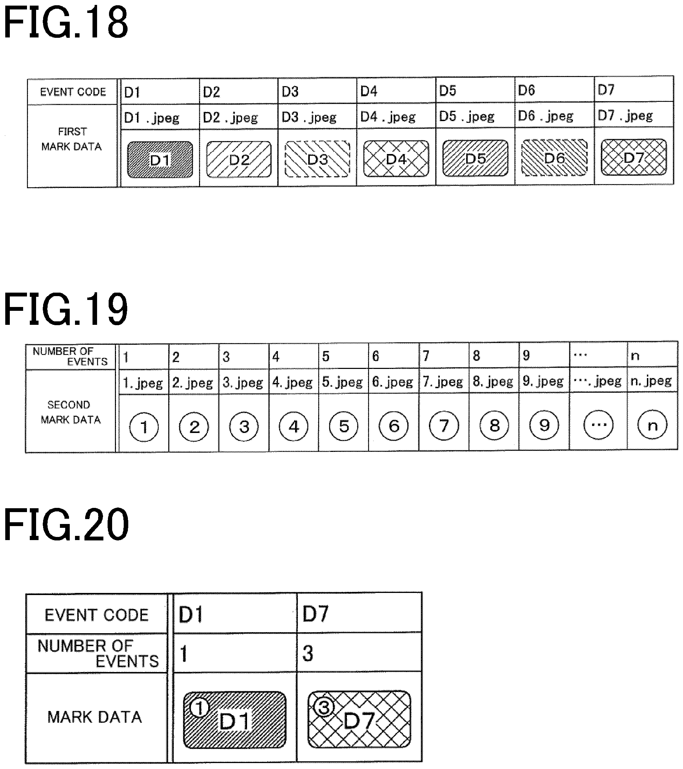

FIG. 18 is a comparison table representing association between event codes and the first mark data used by the received-order management server according to the third embodiment of the present invention in a mark display process.
As illustrated in FIG. 18, the event code and the first mark data are arranged in the vertical direction in the comparison table. In the horizontal direction of the comparison table, D1 to D7 that respectively represent execution dates are arranged as the event codes. Further, D1.jpeg to D7.jpeg that represent data of the respective execution dates are arranged as the first mark data. A jpeg image represented by the identifier "jpeg" is an example, and the first mark data may be a still image having another identifier.

<Number of events and second mark data>

FIG. 19 is a comparison table representing association between the number of events and the second mark data used by the received-order management server according to the third embodiment of the present invention in a mark display process.
As illustrated in FIG. 19, the number of events and the second mark data are arranged in the vertical direction in the comparison table. In the horizontal direction of the comparison table, 1 to n that respectively represent the numbers of times are arranged as the number of events. Further, 1.jpeg to n.jpeg that each represent data of the number of times are arranged as the second mark data.

<Mark data obtained by combining event code and number of events>

FIG. 20 is a comparison table that is an example of the result of a mark display process performed by the received-order management server according to the third embodiment of the present invention, and that represents mark data obtained by combining an event code and the number of events.
As illustrated in FIG. 20, an event code, the number of events, and mark data are arranged in the vertical direction of the comparison table.
In the horizontal direction of the comparison table, D1 and D7 that respectively represent execution dates are arranged as the event codes.
In the horizontal direction of the comparison table, 1 and 3 are arranged as the number of events. Further, the first mark data and the second mark data are superimposed and displayed as the mark data.

<ER diagram>

FIG. 21 is an ER diagram illustrating configurations and association of a management calendar file and a management-calendar event file to be managed by the received-order management server according to the third embodiment of the present invention.
The human-body master M1, the human-body management file F1, and the collection management file F3 illustrated in FIG. 21 are identical to the human-body master M1, the human-body management file F1, and the collection management file F3 illustrated in FIG. 12, and thus descriptions thereof are omitted.

<Management calendar file>

The received-order management server 11 memorizes a customer code, a culture facility code, a management ID, a deletion category, a creation date, a creation time, a creator ID, an update date, an update time, and an updater ID in a management calendar file F21 in association with a human-body ID, a drug ID, and the number of times of production.

The received-order management server 11 extracts the customer code, the culture facility code, the management ID, the deletion category, the creation date, the creation time, the creator ID, the update date, the update time, and the updater ID from the management calendar file F21 by using the human-body ID, the drug ID, and the number of times of production as a key.

<Management calendar event F>

The received-order management server 11 memorizes a date, an event status, a creation date, a creation time, a creator ID, an update date, an update time, and an updater ID in the management calendar event file F23 in association with a human-body ID, a drug ID, the number of times of production, an event code, and the number of events.

The received-order management server 11 extracts the date, the event status, the creation date, the creation time, the creator ID, the update date, the update time, and the updater ID from the management calendar event file F23 by using the human-body ID, the drug ID, the number of times of production, the event code, and the number of events as a key.

<Summary of actions and effects of aspects in present embodiment>

<First aspect>

The received-order management server 11 (a schedule calculation device) according to the present aspect is the received-order management server 11 (the schedule calculation device) that calculates a transportation schedule for a regenerative medicine process related to autologous cells, the transportation schedule including the first transportation process K3 of accommodating a collected material collected from a human body in a container A (a first container) in the medical facility 31 and thereafter transporting the container A (the first container) to the culture facility 35 and the second transportation process K5 of accommodating a drug produced using the collected material in a container B (a second container) different from the container A (the first container) in the culture facility 35 and thereafter transporting the container B (the second container) to the medical facility 31. The received-order management server 11 includes the reception unit 30a that receives administration date data related to a drug specified by a user with respect to calendar screen data for each user displayed on the client terminal 5 (a user terminal) provided in the medical facility 31 from the client terminal 5 (the user terminal), the schedule calculation unit 30b that calculates each of execution dates on which a plurality of processes related to transportation from a collection date to an administration date are respectively performed based on the administration date data received by the reception unit 30a, and the calendar-screen generation unit 30d that generates calendar screen data including the execution dates from the collection date to the administration date.

According to the present aspect, it is possible to calculate each of the execution dates from the collection date to the administration date as the transportation schedule for the regenerative medicine process related to autologous cells, which is related to the first container used for accommodating the collected material collected from a human body and the second container used for accommodating the drug produced using the collected material.

<Second aspect>

The schedule calculation unit 30b according to the present aspect calculates each of the execution dates from the collection date to the administration date by subtracting lead times each of which indicates a time from a certain execution date to another specific execution date, in turn.

According to the present aspect, it is possible to calculate each of the execution dates from the collection date to the administration date by subtracting the lead times each of which indicates the time from a certain execution date to another specific execution date, in turn.

<Third aspect>

The schedule calculation unit 30b according to the present aspect includes the lead-time memory unit 30h that memorizes each of the lead times respectively indicating times related to a plurality of different processes associated in accordance with a specific combination using a customer code specific to the medical facility 31, a drug ID unique to a drug, and a human-body ID unique to a human body. The schedule calculation unit 30b calculates each of the execution dates using each of the lead times acquired from the lead-time memory unit 30h by using, as a key, the specific combination using the customer code specific to the medical facility 31, the drug ID unique to the drug, and the human-body ID unique to the human body received by the reception unit 30a.

According to the present aspect, it is possible to calculate each of the execution dates using each of the lead times acquired from the lead-time memory unit 30h by using, as a key, the specific combination using the customer code specific to the medical facility 31, the drug ID unique to the drug, and the human-body ID unique to the human body.

<Fourth aspect>

The schedule calculation unit 30b according to the present aspect includes the determination unit 30c that determines whether the culture facility 35 can accept administration date data related to culturing the drug. When the determination unit 30c determines that the culture facility 35 can accept the administration date data related to culturing the drug, the calendar-screen generation unit 30d generates calendar screen data.

According to the present aspect, when it is determined that the culture facility 35 can accept the administration date data related to culturing the drug, the calendar-screen generation unit 30d can generate the calendar screen data.

<Fifth aspect>

The determination unit 30c according to the present aspect includes the culture-facility information memory unit 30r that memorizes the number of acceptable cultures of drug in the culture facility 35. The determination unit 30c determines whether there are reservations exceeding the number of acceptable cultures of drug acquired from the culture-facility information memory unit 30r. When it is determined that the reservations do not exceed the number of acceptable cultures of drug, the calendar screen generation unit generates the calendar screen data.

According to the present aspect, when it is determined by the determination unit 30c that the reservations do not exceed the number of acceptable cultures of drug, the calendar screen generation unit can generate the calendar screen data.

<Sixth aspect>

The schedule calculation unit 30b according to the present aspect calculates a processed-product delivery date by subtracting an administration lead time indicating a time from delivery of the drug as a processed product to the medical facility 31 to administration to a human body from the administration date, calculates a processed-product shipping date by subtracting a culture-facility/customer lead time indicating a time of delivery of a collected material from the medical facility 31 to the culture facility 35 from the processed-product delivery date, and calculates a scheduled process completion date by subtracting a processed-product shipping lead time indicating a time from a process completion date to departure of the processed product from the culture facility 35 from the processed-product shipping date.

According to the present aspect, it is possible to calculate the scheduled process completion date by subtracting the processed-product shipping lead time that indicates the time from the process completion date to departure of the processed product from the culture facility 35 from the processed-product shipping date by the schedule calculation unit 30b.

<Seventh aspect>

The schedule calculation unit 30b according to the present aspect includes the drug information memory 30j that memorizes a unit required time required for culturing the drug as a processing lead time, and calculates a collected-material delivery date by subtracting the processing lead time indicating a time from arrival of the collected material at the culture facility 35 to process completion from the scheduled process completion date.

According to the present aspect, it is possible to calculate, by the schedule calculation unit 30b, the collected-material delivery date by subtracting the processing lead time indicating the time from arrival of the collected material at the culture facility 35 to process completion from the scheduled process completion date.

<Eighth aspect>

The schedule calculation unit 30b according to the present aspect calculates a collected-material shipping date by subtracting a culture-facility/customer lead time that indicates a time for delivering the collected material from the medical facility 31 to the culture facility 35 from the collected-material delivery date, and calculates the collection date by subtracting a collected-material shipping lead time indicating a time from a date of collection of the collected material to departure from the medical facility 31, from the collected-material shipping date.

According to the present aspect, it is possible to calculate the collected-material shipping date by subtracting the culture-facility/customer lead time that indicates the time for delivering the collected material from the medical facility 31 to the culture facility 35 from the collected-material delivery date, and to calculate the collection date by subtracting the collected-material shipping lead time indicating the time from the date of collection of the collected material to departure from the medical facility 31 from the collected-material shipping date, by the schedule calculation unit 30b.

<Ninth aspect>

The schedule calculation unit 30b according to the present aspect includes the drug information memory 30j that memorizes the number of cells required for culturing the drug, and the human-body information memory unit 30k that memorizes the number of collectable cells that can be collected from a human body in one collection and a collection interval between at least two or more collection dates related to the human body. The schedule calculation unit 30b sets an integer of a value obtained by dividing the number of cells required for culture acquired from the drug information memory 30j by the number of collectable cells acquired from the human-body information memory unit 30k as the number of times of collection, and calculates the collection dates and the collected-material delivery dates in a case where the number of times of collection is at least two or more based on the number of times of collection and the collection interval acquired from the human-body information memory unit 30k.

According to the present aspect, it is possible to, by the schedule calculation unit 30b, set the integer of the value obtained by dividing the number of cells required for culture by the number of collectable cells as the number of times of collection, and to calculate the collection dates and the collected-material delivery dates in a case where the number of times of collection is at least two or more based on the number of times of collection and the collection interval.

<Tenth aspect>

The calendar-screen generation unit 30d according to the present aspect generates calendar screen data for each user which includes a region for specifying an article, a region for specifying user-identification data, and a region for specifying transportation-destination data.

According to the present aspect, it is possible to generate the calendar screen data for each user which includes the region for specifying an article, the region for specifying user-identification data, and the region for specifying transportation-destination data.

<Eleventh aspect>

The calendar-screen generation unit 30d according to the present aspect includes the mark table 30m that memorizes administration-date mark data, processed-product-delivery-date mark data, processed-product-shipping-date mark data, scheduled-process-completion-date mark data, collected-material-delivery-date mark data, collected-material-shipping-date mark data, and collection-date mark data that are image data representing administration date data, processed-product-delivery date data, processed-product-shipping date data, scheduled-process-completion date data, collected-material-delivery date data, collected-material-shipping date data, and collection date data, respectively, and the mark generation unit 30n that generates the administration-date mark data, the processed-product-delivery-date mark data, the processed-product-shipping-date mark data, the scheduled-process-completion-date mark data, the collected-material-delivery-date mark data, the collected-material-shipping-date mark data, and the collection-date mark data, based on the administration date data, the processed-product-delivery date data, the processed-product-shipping date data, the scheduled-process-completion date data, the collected-material-delivery date data, the collected-material-shipping date data, and the collection date data respectively being represented, by referring to the mark table.

According to the present aspect, it is possible to generate mark data of each execution date based on the data of each execution date by the mark generation unit 30n.

<Twelfth aspect>

The received-order management server 11 (the schedule calculation device) according to the present aspect includes the distribution unit 30g that distributes the administration-date mark data, the processed-product-delivery-date mark data, the processed-product-shipping-date mark data, the scheduled-process-completion-date mark data, the collected-material-delivery-date mark data, the collected-material-shipping-date mark data, and the collection-date mark data that are image data generated by the mark generation unit 30n to the user terminal.

According to the present aspect, it is possible to display each mark data on the user terminal by distributing the administration-date mark data, the processed-product-delivery-date mark data, the processed-product-shipping-date mark data, the scheduled-process-completion-date mark data, the collected-material-delivery-date mark data, the collected-material-shipping-date mark data, and the collection-date mark data generated by the mark generation unit 30n to the user terminal.

<Thirteenth aspect>

The calendar-screen generation unit 30d according to the present aspect includes the management calendar event file F23 that memorizes a date corresponding to each execution date in association with a human-body ID unique to a human body, a drug ID unique to a drug, an event code, and the number of events, the data acquisition unit 30p that acquires the date corresponding to each execution date, the event code, and the number of events from the management calendar event file by using the human-body ID unique to the human body and the drug ID unique to the drug as a key, and the mark display processing unit 30q that superimposes and displays first mark data corresponding to the event code and second mark data corresponding to the number of events in a region corresponding to the date on the calendar screen.

According to the present aspect, it is possible to superimpose and display the first mark data corresponding to the event code and the second mark data corresponding to the number of events in the region corresponding to the date on the calendar screen by the mark display processing unit 30q.

<Fourteenth aspect>

The schedule management system 1 according to the present aspect includes the received-order management server 11 (the schedule calculation device) according to any one of the first to thirteenth aspects and the client terminal 5 (the user terminal).

According to the present aspect, it is possible to provide the schedule management system 1 including the client terminal 5.

<Fifteenth aspect>

A schedule calculation method according to the present aspect is a schedule calculation method by the received-order management server 11 (the schedule calculation device) that calculates a transportation schedule for a regenerative medicine process related to autologous cells, the transportation schedule including the first transportation process K3 of accommodating a collected material collected from a human body in a container A (a first container) in the medical facility 31 and thereafter transporting the container A (the first container) to the culture facility 35 and the second transportation process K5 of accommodating a drug produced using the collected material in a second container different from the container A (the first container) in the culture facility 35 and thereafter transporting the second container to the medical facility 31. The method performs a reception step (Step S207) that receives administration date data related to a drug specified by a user with respect to calendar screen data for each user displayed on the client terminal 5 (a user terminal) provided in the medical facility 31 from the client terminal 5 (the user terminal), a schedule calculation step (Steps S213 to S225) that calculates each of execution dates on which a plurality of processes related to transportation from a collection date to an administration date are respectively performed based on the administration date data received at the reception step, and a calendar screen generation step (Steps S401 to S417) that generates calendar screen data including the execution dates from the collection date to the administration date.

Actions and effects of the fifteenth aspect are identical to those of the first aspect, and thus descriptions thereof are omitted.

Reference Signs List

N1, N2, N3 communication network, 1 schedule management system, 3 client terminal, 5 client terminal, 7 received-order management device, 9 front-end server, 11 received-order management server, 13 logistics management server, 15 database DB, 17 transportation unit, 21 vehicle, 26 collection container, 27 small terminal, 27a CPU, 27b ROM, 27c RAM, 27d operation display unit, 27e GPS receiver, 27f axis sensor, 27g communication unit, 27h communication unit, 30 controller, 30a reception unit, 30b schedule calculation unit, 30c determination unit, 30d calendar-screen generation unit, 30g distribution unit, 30h lead-time memory unit, 30i production-schedule memory unit, 30j drug information memory unit, 30k human-body information memory unit, 30m mark table, 30n mark generation unit, 30p data acquisition unit, 30q mark display processing unit, 30r culture-facility information memory unit, 31 medical facility, 33 logistics center, 35 culture facility, 37 user

**Claims**

1. A schedule calculation device for calculating a transportation schedule for a regenerative medicine process related to autologous cells, the transportation schedule including a first transportation process that accommodates a collected material collected from a human body in a first container in a medical facility and thereafter transports the first container to a culture facility and a second transportation process that accommodates a drug produced using the collected material in a second container different from the first container in a culture facility and thereafter transports the second container to the medical facility, the device comprising:

a reception unit that receives administration date data related to the drug specified by a user with respect to calendar screen data for each user displayed on a user terminal provided in the medical facility, from the user terminal;

a schedule calculation unit that calculates each of execution dates on which a plurality of processes related to transportation from a collection date to the administration date are respectively performed based on the administration date data received by the reception unit; and

a calendar-screen generation unit that generates calendar screen data including the execution dates from the collection date to the administration date.

2. The schedule calculation device according to claim 1, wherein the schedule calculation unit calculates the execution dates from the collection date to the administration date by subtracting lead times each representing a time from a certain execution date to another specific execution date in turn.

3. The schedule calculation device according to claim 1, wherein

the schedule calculation unit includes a lead-time memory unit that memorizes each of lead times that represent times related to a plurality of different processes associated in accordance with a specific combination using a customer code specific to the medical facility, a drug ID unique to the drug, and a human-body ID unique to the human body, and

the schedule calculation unit calculates each execution date by using each lead time acquired from the lead-time memory unit by using a specific combination that uses a customer code specific to the medical facility, a drug ID unique to the drug, and a human-body ID unique to the human body received by the reception unit as a key.

4. The schedule calculation device according to claim 1, wherein

the schedule calculation unit includes a determination unit that determines whether the culture facility is able to accept the administration date data related to culture of the drug, and

when it is determined by the determination unit that the culture facility is able to accept the administration date data related to culture of the drug, the calendar-screen generation unit generates the calendar screen data.

5. The schedule calculation device according to claim 4, wherein

the determination unit includes a culture-facility information memory unit that memorizes number of acceptable cultures of drug in the culture facility, and

the determination unit determines whether there are reservations exceeding the number of acceptable cultures of drug acquired from the culture-facility information memory unit, and the calendar-screen generation unit generates the calendar screen data when it is determined that the reservations do not exceed the number of acceptable cultures of drug.

6. The schedule calculation device according to claim 1, wherein the schedule calculation unit calculates a processed-product delivery date by subtracting an administration lead time that represents a time from delivery of a drug as a processed product to the medical facility to administration to a human body, from the administration date,

calculates a processed-product shipping date by subtracting a culture-facility/customer lead time that represents a time of delivery of the collected material from the medical facility to the culture facility, from the processed-product delivery date, and

calculates a scheduled process completion date by subtracting a processed-product shipping lead time that represents a time from a process completion date to departure of a processed product from the culture facility from the processed-product shipping date.

7. The schedule calculation device according to claim 6, wherein

the schedule calculation unit includes a drug information memory unit that memorizes a unit required time required for culturing the drug as a processing lead time, and
the schedule calculation unit calculates a collected-material delivery date by subtracting the processing lead time that represents a time from arrival of the collected material at the culture facility to process completion, from the scheduled process completion date.

8. The schedule calculation device according to claim 7, wherein the schedule calculation unit calculates a collected-material shipping date by subtracting a culture-facility/customer lead time that represents a time of delivery of the collected material from a medical facility to a culture facility from the collected-material delivery date, and calculates a collection date by subtracting a collected-material shipping lead time that represents a time from a date of collection of the collected material to departure of the collected material from the medical facility from the collected-material shipping date.

9. The schedule calculation device according to claim 7, wherein

the schedule calculation unit includes
a drug information memory unit that memorizes number of cells required for culture of the drug, and
a human-body information memory unit that memorizes number of cells collectable from the human body in one collection and a collection interval between at least two or more collection dates related to the human body, and
the schedule calculation unit sets an integer of a value obtained by dividing the number of cells required for culture acquired from the drug information memory unit by the number of collectable cells acquired from the human-body information memory unit as number of times of collection, and calculates the collection dates and the collected-material delivery dates in a case where the number of times of collection is at least two or more based on the number of times of collection and the collection interval acquired from the human-body information memory unit.

10. The schedule calculation device according to claim 1, wherein the calendar-screen generation unit generates the calendar screen data for each user including a region for specifying the article, a region for specifying user-identification data, and a region for specifying the transportation-destination data.

11. The schedule calculation device according to claim 1, wherein the calendar-screen generation unit includes

a mark table that memorizes administration-date mark data, processed-product-delivery-date mark data, processed-product-shipping-date mark data, scheduled-process-completion-date mark data, collected-material-delivery-date mark data, collected-material-shipping-date mark data, and collection-date mark data that are image data representing the administration date data, processed-product-delivery date data, processed-product-shipping date data, scheduled-process-completion date data, collected-material-delivery date data, collected-material-shipping date data, and collection date data, respectively, and
a mark generation unit that generates administration-date mark data, processed-product-delivery-date mark data, processed-product-shipping-date mark data, scheduled-process-completion-date mark data, collected-material-delivery-date mark data, collected-material-shipping-date mark data, and collection-date mark data based on the administration date data, the processed-product-delivery date data, the processed-product-shipping date data, the scheduled-process-completion date data, collected-material-delivery date data, the collected-material-shipping date data, and the collection date data respectively being represented, by referring to the mark table.

12. The schedule calculation device according to claim 11, further comprising a distribution unit that distributes the administration-date mark data, the processed-product-delivery-date mark data, the processed-product-shipping-date mark data, the scheduled-process-completion-date mark data, collected-material-delivery-date mark data, the collected-material-shipping-date mark data, and the collection-date mark data that are image data generated by the mark generation unit, to the user terminal.

13. The schedule calculation device according to claims 1, 10, and 11, wherein the calendar-screen generation unit includes

a management calendar event file that memorizes a date corresponding to each execution date in association with a human-body ID unique to the human body, a drug ID unique to the drug, an event code, and number of events,

a data acquisition unit that acquires a date, an event code, and number of events from a management calendar event file by using the human-body ID and the drug ID as a key, and
a mark display processing unit that superimposes and displays first mark data corresponding to the event code and second mark data corresponding to the number of events in a region corresponding to a date on a calendar screen.

14. A schedule management system comprising:

the schedule calculation device according to any one of claims 1 to 13; and
a user terminal.

15. A schedule calculation method by a schedule calculation device that calculates a transportation schedule for a regenerative medicine process related to autologous cells, the transportation schedule including a first transportation process that accommodates a collected material collected from a human body in a first container in a medical facility and thereafter transports the first container to a culture facility and a second transportation process that accommodates a drug produced using the collected material in a second container different from the first container in a culture facility and thereafter transports the second container to the medical facility, the method comprising:

a reception step that receives administration date data related to the drug specified by the user with respect to calendar screen data for each user displayed on a user terminal provided in the medical facility, from the user terminal;
a schedule calculation step that calculates each of execution dates on which a plurality of processes related to transportation from a collection date to the administration date are respectively performed based on the administration date data received at the reception step; and
a calendar screen generation step that generates calendar screen data including the execution dates from the collection date to the administration date.

# FIG.1

# FIG.2

# FIG.3

Se3 OPENING/ CLOSING SENSOR

Se2 HYGROMETER

Se1 THERMOMETER

26A COLLECTION CONTAINER

26B COLLECTION CONTAINER

TRANSPORTATION UNIT

17

27h COMMUNICATION UNIT

27g COMMUNICATION UNIT

ANT1

27a CPU

27c RAM

27b ROM

27d OPERATION DISPLAY UNIT

27f TEN-AXIS SENSOR

27e GPS RECEIVER

ANT2

SMALL TERMINAL

27

# FIG.4

K1

## REGENERATIVE MEDICINE PROCESS

**31**

### MEDICAL FACILITY

5

#### CLIENT TERMINAL

FIRST TRANSPORTATION PROCESS — K3

SECOND TRANSPORTATION PROCESS — K5

**35**

### CULTURE FACILITY

3

#### CLIENT TERMINAL

30

## CONTROLLER

30a — RECEPTION UNIT

DISTRIBUTION UNIT — 30g

30f — RECEPTION UNIT

DISTRIBUTION UNIT — 30e

SCHEDULE CALCULATION UNIT — 30b

HUMAN-BODY INFORMATION MEMORY UNIT — 30k

DRUG INFORMATION MEMORY UNIT — 30j

LEAD-TIME MEMORY UNIT — 30h

DETERMINATION UNIT — 30c

PRODUCTION-SCHEDULE MEMORY UNIT — 30i

CULTURE-FACILITY INFORMATION MEMORY UNIT — 30r

CALENDAR-SCREEN GENERATION UNIT — 30d

MARK TABLE — 30m

MARK GENERATION UNIT — 30n

DATA ACQUISITION UNIT — 30p

MARK DISPLAY PROCESSING UNIT — 30q

# FIG.5

MEDICAL FACILITY    31

LOGISTICS CENTER    33

CULTURE FACILITY    35

S11 DELIVER CONTAINER A (EMPTY)

S13 STORE CELLS IN CONTAINER A

S15 DELIVER CONTAINER A (ACCOMMODATING CELLS)

S17 PROCESS CELLS

S19 DELIVER CONTAINER B (EMPTY)

S21 STORE PROCESSED MATERIAL IN CONTAINER B

S23 DELIVER CONTAINER B (ACCOMMODATING PROCESSED MATERIAL)

S25 COLLECT CONTAINER A (EMPTY)

S27 COLLECT CONTAINER B (EMPTY)

30

# FIG.6

DATA ——————————⟶
MATERIAL - - - - - ⟶

| 13 | 11 | 17 | 31 | 35 |
|---|---|---|---|---|
| LOGISTICS MANAGEMENT SERVER | RECEIVED-ORDER MANAGEMENT SERVER | TRANSPORTATION UNIT (CONTAINER A) | MEDICAL FACILITY | CULTURE FACILITY |

S41
DISPLAY PERSONAL CALENDAR

S43
REGISTER ADMINISTRATION DATE

S45
CHECK WHETHER ADMINISTRATION DATE IS REGISTRABLE

S47
CALCULATE EACH EXECUTION DATE

S49
DISPLAY CALENDAR INCLUDING EACH EXECUTION DATE

S51
ASSOCIATE RECEIVED-ORDER DATA

S53
CREATE TRANSPORTATION INFORMATION

S55
FORWARD TRANSPORTATION INFORMATION

S57
TRANSPORT EMPTY CONTAINER

S59
UPDATE STATUS (INFORMATION INDICATING THAT EMPTY CONTAINER HAS BEEN TRANSPORTED)

S61
STORE CELLS

S63
UPDATE STATUS

S65
MONITOR (TEMPERATURE, FOR EXAMPLE)

S67
COLLECT CONTAINER ON CALCULATED COLLECTED-MATERIAL SHIPPING DATE

S69
COLLECT CONTAINER AND ISSUE RECEIPT CERTIFICATE

S71
UPDATE STATUS

S73
ASSOCIATE COLLECTION DATA

S75
CREATE TRANSPORTATION INFORMATION

S77
FORWARD TRANSPORTATION INFORMATION

S79
DELIVER CONTAINER AND ISSUE HANDOVER CERTIFICATE

S81
UPDATE STATUS

# FIG.7

DATA ———→
MATERIAL - - - - →

| 13 | 11 | 17 | 35 | 31 |
|---|---|---|---|---|
| LOGISTICS MANAGEMENT SERVER | RECEIVED-ORDER MANAGEMENT SERVER | TRANSPORTATION UNIT (CONTAINER B) | CULTURE FACILITY | MEDICAL FACILITY |

S101
DISPLAY PERSONAL CALENDAR

S103
REGISTER PROCESS COMPLETION DATE

S105
CALCULATE EACH EXECUTION DATE

S107
DISPLAY CALENDAR INCLUDING EACH OF UPDATED EXECUTION DATES

S109
ASSOCIATE PROCESSED-PRODUCT DELIVERY INFORMATION

S111
CREATE TRANSPORTATION INFORMATION

S113
FORWARD TRANSPORTATION INFORMATION

S115
TRANSPORT EMPTY CONTAINER

S117
UPDATE STATUS (INFORMATION INDICATING THAT EMPTY CONTAINER HAS BEEN TRANSPORTED)

S119
STORE PROCESSED MATERIAL

S121
UPDATE STATUS

S123
監視(温度等)

S125
COLLECT CONTAINER ON CALCULATED PROCESSED-PRODUCT SHIPPING DATE

S127
COLLECT CONTAINER AND ISSUE RECEIPT CERTIFICATE

S129
UPDATE STATUS

S131
COLLECT CONTAINER ON CALCULATED PROCESSED-PRODUCT SHIPPING DATE

S133
DELIVER CONTAINER AND ISSUE DELIVERY CERTIFICATE

S135
UPDATE STATUS

S137
PERFORM ADMINISTRATION

S139
UPDATE STATUS

S141
COLLECT CONTAINER ON CALCULATED CONTAINER COLLECTION DATE

S143
COLLECT CONTAINER

S145
UPDATE STATUS

32

# FIG.8

(a)

| SUN | MON | TUS | WED | THU | FRI | SAT |
|---|---|---|---|---|---|---|
| | 1 | 2 | 3 | 4 | 5 | 6 |
| 7 | 8 | 9 | 10 | 11 | 12 G7 D7 | 13 G6 D6 |
| 14 | 15 | 16 | 17 G5 D5 | 18 | 19 | 20 |
| 21 | 22 G2 | 23 G1 | 24 D4 | 25 D3 | 26 | 27 |
| 28 | 29 D2 | 30 D1 | 31 G4 | G3 | | |

(b)

| SIGN | D1 | D2 | D3 | D4 | D5 | D6 | D7 |
|---|---|---|---|---|---|---|---|
| NAME | ADMINISTRATION DATE | PROCESSED-PRODUCT DELIVERY DATE | PROCESSED-PRODUCT SHIPPING DATE | SCHEDULED PROCESS COMPLETION DATE | COLLECTED-MATERIAL DELIVERY DATE | COLLECTED-MATERIAL SHIPPING DATE | COLLECTION DATE |
| MARK DATA | D1 | D2 | D3 | D4 | D5 | D6 | D7 |

# FIG.9

37
USER

5
CLIENT TERMINAL
(MEDICAL FACILITY)

11
RECEIVED-ORDER MANAGEMENT
SERVER (ADMINISTRATION
INFORMATION IS REGISTERED)

S201
PERFORM OPERATION OF SELECTING
CALENDAR SCREEN

S203
START UP CALENDAR SCREEN

S205
PERFORM OPERATION OF
REGISTERING RECEIVED-ORDER DATA

S207
TRANSMIT ADMINISTRATION DATE,
PRODUCT CODE, CUSTOMER CODE,
AND HUMAN-BODY CODE

S209
CHECK WHETHER
ADMINISTRATION DATE IS
REGISTRABLE

ERROR IN INPUT CHECK

S212
DISPLAY MESSAGE

S211
TRANSMIT ERROR MESSAGE

S213
CALCULATE PROCESSED-
PRODUCT DELIVERY DATE

S215
CALCULATE PROCESSED-
PRODUCT SHIPPING DATE

S217
CALCULATE SCHEDULED
PROCESS COMPLETION DATE

S219
CALCULATE COLLECTED-
MATERIAL DELIVERY DATE

S221
CALCULATE COLLECTED-
MATERIAL SHIPPING DATE

S223
CALCULATE
COLLECTION DATE

S225
CALCULATE ADVANCE
TRANSPORTATION DATE

S227
GENERATE
CALENDAR SCREEN

S229
TRANSMIT RESULT

# FIG.10

41

| | CALCULATED DATE | PARENT ITEM | CHILD ITEM | OPERATOR |
|---|---|---|---|---|
| 1 | PROCESSED-PRODUCT DELIVERY DATE D2 | ADMINISTRATION DATE D1 | ADMINISTRATION LEAD TIME T1 | SUBTRACTION – |
| 2 | PROCESSED-PRODUCT SHIPPING DATE D3 | PROCESSED-PRODUCT DELIVERY DATE D2 | CPC/CUSTOMER LEAD TIME T2 | SUBTRACTION – |
| 3 | SCHEDULED PROCESS COMPLETION DATE D4 | PROCESSED-PRODUCT SHIPPING DATE D3 | PROCESSED-PRODUCT SHIPPING LEAD TIME T3 | SUBTRACTION – |
| 4 | COLLECTED-MATERIAL DELIVERY DATE D5 | SCHEDULED PROCESS COMPLETION DATE D4 | PROCESSING LEAD TIME T4 | SUBTRACTION – |
| 5 | COLLECTED-MATERIAL SHIPPING DATE D6 | COLLECTED-MATERIAL DELIVERY DATE D5 | CPC/CUSTOMER LEAD TIME T2 | SUBTRACTION – |
| 6 | COLLECTION DATE D7 | COLLECTED-MATERIAL SHIPPING DATE D6 | COLLECTED-MATERIAL SHIPPING LEAD TIME T5 | SUBTRACTION – |

# FIG.11

(a)

START

ACQUIRE TARGET DATA FROM CALCULATION FILE ~S301

REPEAT PROCESS FOR NUMBER OF CALCULATION CATEGORIES ~S303

CALCULATE CALCULATED DATE
CALCULATED DATE = PARENT ITEM OPERATOR (+, –) CHILD ITEM ~S305

REGISTER CALCULATED DATE IN MASTER OF REGISTRATION DESTINATION ~S307

HAS CONDITION FOR ENDING LOOP BEEN SATISFIED? ~S309

No

Yes

END

(b)

43

CALCULATION FILE

HUMAN-BODY ID

MANAGEMENT ID

CALCULATION CATEGORY
(CATEGORY OF EACH DATE DISPLAYED IN CALENDAR)

PARENT ITEM

CHILD ITEM

OPERATOR

REGISTRATION DESTINATION

# FIG.13

| SUN | MON | TUS | WED | THU | FRI | SAT |
|---|---|---|---|---|---|---|
| | 1<br>G71—①D7 | 2<br>①D6 | 3<br>—G61 | 4 | 5<br>①D5 | 6<br>—G51 |
| 7 | 8<br>G72—②D7 | 9<br>②D6 | 10<br>—G62 | 11 | 12<br>②D5 | 13<br>—G52 |
| 14 | 15<br>G73—③D7 | 16<br>③D6 | 17<br>—G63 | 18 | 19<br>③D5 | 20<br>—G53 |
| 21 | 22 | 23 | 24 | 25<br>G4—D4 | 26<br>D3—G3 | 27 |
| 28 | 29<br>G2—D2 | 30<br>D1—G1 | 31 | | | |

# FIG.14

```
        37                      5                           11
┌──────────────┐    ┌──────────────┐    ┌──────────────────────────┐
│              │    │CLIENT TERMINAL│    │  RECEIVED-ORDER           │
│    USER      │    │ (MEDICAL     │    │ MANAGEMENT SERVER         │
│              │    │   FACILITY)  │    │ (ADMINISTRATION           │
└──────────────┘    └──────────────┘    │ INFORMATION IS REGISTERED)│
                                         └──────────────────────────┘
```

S201
PERFORM OPERATION OF
SELECTING CALENDAR SCREEN

S203
START UP CALENDAR SCREEN

S205
PERFORM OPERATION OF
REGISTERING RECEIVED-ORDER
DATA

S207
TRANSMIT ADMINISTRATION
DATE, PRODUCT CODE,
CUSTOMER CODE, AND HUMAN-
BODY CODE

S209
CHECK WHETHER
ADMINISTRATION DATE IS
REGISTRABLE

ERROR IN INPUT CHECK

S212
DISPLAY MESSAGE

S211
TRANSMIT ERROR MESSAGE

S213
CALCULATE PROCESSED-
PRODUCT DELIVERY DATE

S215
CALCULATE PROCESSED-
PRODUCT SHIPPING DATE

S217
CALCULATE SCHEDULED
PROCESS COMPLETION DATE

S351
CALCULATE NUMBER OF
TIMES OF COLLECTION

S219
CALCULATE COLLECTED-
MATERIAL DELIVERY DATE

S221
CALCULATE COLLECTED-
MATERIAL SHIPPING DATE

S223
CALCULATE COLLECTION DATE

REPEAT PROCESSES FOR
(NUMBER OF TIMES OF
COLLECTION-1) TIMES

S353
CALCULATE
COLLECTION DATE

S355
CALCULATE
COLLECTED-MATERIAL
SHIPPING DATE

S357
CALCULATE
COLLECTED-MATERIAL
DELIVERY DATE

S225
CALCULATE ADVANCE
TRANSPORTATION DATE

S227
GENERATE CALENDAR SCREEN

S229
TRANSMIT RESULT

# FIG.15

| | CALCULATED DATE | PARENT ITEM | CHILD ITEM | OPERATOR |
|---|---|---|---|---|
| 1 | PROCESSED-PRODUCT DELIVERY DATE D2 | ADMINISTRATION DATE D1 | ADMINISTRATION LEAD TIME T1 | SUBTRACTION — |
| 2 | PROCESSED-PRODUCT SHIPPING DATE D3 | PROCESSED-PRODUCT DELIVERY DATE D2 | CPC/CUSTOMER LEAD TIME T2 | SUBTRACTION — |
| 3 | SCHEDULED PROCESS COMPLETION DATE D4 | PROCESSED-PRODUCT SHIPPING DATE D3 | PROCESSED-PRODUCT SHIPPING LEAD TIME T3 | SUBTRACTION — |
| 4 | COLLECTED-MATERIAL DELIVERY DATE D5[1] | SCHEDULED PROCESS COMPLETION DATE D4 | PROCESSING LEAD TIME T4 | SUBTRACTION — |
| 5 | COLLECTED-MATERIAL SHIPPING DATE D6[1] | COLLECTED-MATERIAL DELIVERY DATE D5[1] | CPC/CUSTOMER LEAD TIME T2 | SUBTRACTION — |
| 6 | COLLECTION DATE D7[1] | COLLECTED-MATERIAL SHIPPING DATE D6[1] | COLLECTED-MATERIAL SHIPPING LEAD TIME T5 | SUBTRACTION — |
| 7 | SECOND COLLECTION DATE D7[2] | THIRD COLLECTION DATE D7[3] | COLLECTION INTERVAL LEAD TIME T6 | SUBTRACTION — |
| 8 | SECOND COLLECTED-MATERIAL SHIPPING DATE D6[2] | SECOND COLLECTION DATE D7[2] | COLLECTED-MATERIAL SHIPPING LEAD TIME T5 | ADDITION + |
| 9 | SECOND COLLECTED-MATERIAL DELIVERY DATE D5[2] | SECOND COLLECTED-MATERIAL SHIPPING DATE D6[2] | CPC/CUSTOMER LEAD TIME T2 | ADDITION + |
| 10 | FIRST COLLECTION DATE D7[1] | SECOND COLLECTION DATE D7[2] | COLLECTION INTERVAL LEAD TIME T6 | SUBTRACTION — |
| 11 | FIRST COLLECTED-MATERIAL SHIPPING DATE D6[1] | FIRST COLLECTION DATE D7[1] | COLLECTED-MATERIAL SHIPPING LEAD TIME T5 | ADDITION + |
| 12 | FIRST COLLECTED-MATERIAL DELIVERY DATE D5[1] | FIRST COLLECTED-MATERIAL SHIPPING DATE D6[1] | CPC/CUSTOMER LEAD TIME T2 | ADDITION + |

# FIG.16

START

S401 — ACQUIRE CUSTOMER CODE, DRUG ID, AND HUMAN-BODY ID FROM PERSONAL CALENDAR SCREEN

S403 — CALCULATE TARGET RANGE PERIOD OF EVENTS TO BE ACQUIRED

S405 — REPEAT PROCESSES FOR NUMBER OF RECORDS IN TARGET RANGE PERIOD

S407 — ACQUIRE RECORD CORRESPONDING TO EACH EXECUTION DATE FROM MANAGEMENT CALENDAR EVENT F BY USING HUMAN-BODY ID AND DRUG ID AS KEY

S409 — ACQUIRE DATE

S411 — IS DATE WITHIN TARGET RANGE PERIOD ? — No

Yes

S413 — ACQUIRE EVENT CODE AND NUMBER OF EVENTS

S415 — MARK DISPLAY PROCESS

S417 — HAS LOOP ENDED?

No

Yes

END

# FIG.17

S450 — ( PERFORM MARK DISPLAY PROCESS )

S451 — READ FIRST MARK DATA CORRESPONDING TO EVENT CODE

S453 — READ SECOND MARK DATA CORRESPONDING TO NUMBER OF EVENTS

S455 — SUPERIMPOSE AND DISPLAY FIRST MARK DATA AND SECOND MARK DATA IN REGION CORRESPONDING TO DATE

( RETURN )

# FIG.18

| EVENT CODE | D1 | D2 | D3 | D4 | D5 | D6 | D7 |
|---|---|---|---|---|---|---|---|
| FIRST MARK DATA | D1 . jpeg | D2 . jpeg | D3 . jpeg | D4 . jpeg | D5 . jpeg | D6 . jpeg | D7 . jpeg |
| | D1 | D2 | D3 | D4 | D5 | D6 | D7 |

# FIG.19

| NUMBER OF EVENTS | 1 | 2 | 3 | 4 | 5 | 6 | 7 | 8 | 9 | ··· | n |
|---|---|---|---|---|---|---|---|---|---|---|---|
| SECOND MARK DATA | 1. jpeg | 2. jpeg | 3. jpeg | 4. jpeg | 5. jpeg | 6. jpeg | 7. jpeg | 8. jpeg | 9. jpeg | ···. jpeg | n. jpeg |
| | 1 | 2 | 3 | 4 | 5 | 6 | 7 | 8 | 9 | ··· | n |

# FIG.20

| EVENT CODE | D1 | D7 |
|---|---|---|
| NUMBER OF EVENTS | 1 | 3 |
| MARK DATA | ① D1 | ③ D7 |

FIG.21

EP 3 926 640 A1

F21

MANAGEMENT CALENDAR F

HUMAN-BODY ID
DRUG ID
NUMBER OF TIMES
  OF PRODUCTION

CUSTOMER CODE

CULTURE FACILITY CODE

MANAGEMENT ID

DELETION CATEGORY

CREATION DATE

CREATION TIME

CREATOR ID

UPDATE DATE

UPDATE TIME

UPDATER ID

F23

MANAGEMENT CALENDAR EVENT F

HUMAN-BODY ID
DRUG ID
NUMBER OF TIMES
  OF PRODUCTION
EVENT CODE
NUMBER OF EVENTS

DATE

EVENT STATUS

CREATION DATE

CREATION TIME

CREATOR ID

UPDATE DATE

UPDATE TIME

UPDATER ID

M1

HUMAN-BODY M

HUMAN-
BODY ID

SEX
WEIGHT
HEIGHT
AGE

F1

HUMAN-BODY
MANAGEMENT F

HUMAN-BODY ID
DRUG ID
MANAGEMENT ID
CUSTOMER CODE

COLLECTION
MANAGEMENT ID

PROCESSING
MANAGEMENT ID

ADMINISTRATION
MANAGEMENT ID

F3

COLLECTION MANAGEMENT F

COLLECTION
MANAGEMENT ID
TRANSPORTATION-UNIT
                   ID

DRUG ID

TYPE OF COLLECTED MATERIAL

COLLECTED-MATERIAL SHIPPING
LEAD TIME

COLLECTION INTERVAL LEAD TIME

AMOUNT OF COLLECTION IN ONE
COLLECTION

COLLECTION-CONTAINER ID (1)

COLLECTION-CONTAINER ID (2)
...

## INTERNATIONAL SEARCH REPORT

| International application No. |
|---|
| PCT/JP2019/004830 |

### A. CLASSIFICATION OF SUBJECT MATTER
Int. Cl. G16H40/20(2018.01)i, G06Q10/02(2012.01)i, G06Q10/08(2012.01)i

According to International Patent Classification (IPC) or to both national classification and IPC

### B. FIELDS SEARCHED

Minimum documentation searched (classification system followed by classification symbols)
Int. Cl. G16H40/20, G06Q10/02, G06Q10/08

Documentation searched other than minimum documentation to the extent that such documents are included in the fields searched
Published examined utility model applications of Japan    1922-1996
Published unexamined utility model applications of Japan   1971-2019
Registered utility model specifications of Japan          1996-2019
Published registered utility model applications of Japan   1994-2019

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2003-108660 A (EGAWA, Kouji) 11 April 2003, paragraphs [0001]-[0083], fig. 1-20 (Family: none) | 1-8, 10-15 |
| Y | JP 2018-136631 A (MEDIPAL HOLDINGS CORPORATION) 30 August 2018, paragraphs [0010]-[0071], fig. 1-17 & WO 2018/150855 A1, paragraphs [0010]-[0071], fig. 1-17 | 1-8, 10-15 |
| A | JP 2005-284414 A (JMS CO., LTD.) 13 October 2005, entire text, all drawings (Family: none) | 1-15 |
| A | JP 2006-4299 A (HITACHI MEDICAL CORP.) 05 January 2006, entire text, all drawings (Family: none) | 1-15 |

☒  Further documents are listed in the continuation of Box C.      ☐  See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10.05.2019 | 21.05.2019 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2019/004830

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2006-259993 A (SEIKO EPSON CORPORATION) 28 September 2006, entire text, all drawings (Family: none) | 1-15 |

Form PCT/ISA/210 (continuation of second sheet) (January 2015)

**EP 3 926 640 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6275388 B